(19) Europäisches Patentamt
European Patent Office
Office européen des brevets

(11) EP 1 792 642 A2

(12) DEMANDE DE BREVET EUROPEEN

(43) Date de publication:
**06.06.2007 Bulletin 2007/23**

(21) Numéro de dépôt: **06301193.6**

(22) Date de dépôt: **30.11.2006**

(51) Int Cl.:
***A61Q 19/08*** *(2006.01)* ***A61K 8/06*** *(2006.01)*
***A61K 8/81*** *(2006.01)*

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **01.12.2005 FR 0553680**

(71) Demandeur: **L'ORÉAL**
**75008 Paris (FR)**

(72) Inventeurs:
- **Cassin, Guillaume**
  **91140, Villebon sur Yvette (FR)**
- **Gourlaouen, Véronique**
  **91320, Wissous (FR)**

(74) Mandataire: **Le Coupanec, Pascale A.M.P.**
**Nony & Associés,**
**3 rue de Penthièvre**
**75008 Paris (FR)**

# (54) Composition cosmétique contenant un polymère statistique à chaîne principale linéaire de nature éthylénique

(57) La présente invention concerne une composition cosmétique comprenant, dans un milieu physiologiquement acceptable, au moins un copolymère statistique à chaîne principale linéaire de nature éthylénique, dans laquelle ledit copolymère présente une masse moléculaire comprise entre 15 000 et 600 000 g/mol, contient au moins 70 % d'unités monomères issues de monomères dont les homopolymères sont hydrophobes et présentent une température de transition vitreuse supérieure à 40 °C, contient également au moins une « unité monomère » issue d'un monomère hydrophile ionique au moins partiellement neutralisée, et présente une température globale de transition vitreuse supérieure ou égale à 45 °C et se présentant sous la forme d'une émulsion eau-dans-huile ou d'une émulsion multiple.

EP 1 792 642 A2

## Description

La présente invention se rapporte à une composition antirides contenant au moins un copolymère statistique à chaîne principale linéaire de nature éthylénique caractérisée en ce qu'elle se présente sous la forme d'une émulsion eau-dans-huile ou d'une émulsion multiple, et à l'utilisation de ce copolymère comme agent tenseur dans une composition cosmétique sous forme d'émulsion eau-dans-huile ou d'émulsion multiple, destinées notamment au traitement, à la diminution, à l'effacement et/ou au lissage des rides et ridules de la peau de l'être humain.

Au cours du processus de vieillissement, il apparaît différents signes sur la peau, très caractéristiques de ce vieillissement, se traduisant notamment par une modification de la structure et des fonctions cutanées. Les principaux signes cliniques du vieillissement cutané sont notamment l'apparition de ridules et de rides profondes, en augmentation avec l'âge. On constate en particulier une désorganisation du « grain » de la peau, c'est-à-dire que le micro-relief est moins régulier et présente un caractère anisotrope.

Il est connu de traiter ces signes du vieillissement en utilisant des compositions cosmétiques ou dermatologiques contenant des actifs capables de lutter contre le vieillissement, tels que les $\alpha$-hydroxy-acides, les $\beta$-hydroxy-acides et les rétinoïdes. Ces actifs agissent sur les rides en éliminant les cellules mortes de la peau et en accélérant le processus de renouvellement cellulaire. Toutefois, ces actifs présentent l'inconvénient de n'être efficaces pour le traitement des rides qu'après un certain temps d'application. Or, on cherche de plus en plus à obtenir un effet immédiat des actifs utilisés, conduisant rapidement à un lissage des rides et ridules et à la disparition des marques de fatigue.

La présente invention a justement pour objet l'utilisation, en émulsion eau-dans-huile ou multiple, d'un copolymère particulier permettant d'obtenir immédiatement cet effet.

Il est connu d'utiliser dans des compositions cosmétiques des polymères synthétiques comme agents tenseurs.

On connaît ainsi de WO 98/29092 une composition à effet tenseur comprenant une dispersion aqueuse d'un système polymérique contenant au moins un polymère d'origine synthétique présentant un poids moléculaire supérieur à 670 000 g/mol, choisi parmi différents types de polyuréthannes, les polyurées, les polymères ou copolymères acryliques, les polymères d'acide isophtalique sulfoné et leurs mélanges. Le toucher cosmétique de ces compositions n'est cependant pas toujours satisfaisant.

On connaît également de EP 1 038 519 l'utilisation de certains polymères siliconés spécifiques pour jouer le rôle d'agents à effet tenseur, ou encore de WO 00/30595 des copolymères contenant en poids de 20 à 90 % d'un lactame vinylique, de 1 à 55 % d'acide carboxylique polymérisable et de 1 à 25 % d'un monomère hydrophobe tel qu'un acrylate ou méthacrylate d'alkyle en $C_{10}$ à $C_{24}$ permettant de fournir des gels mous utilisables dans des compositions cosmétiques antirides.

Enfin, le document FR 2 843 025 décrit l'utilisation de réseaux interpénétrés de polymères en tant qu'agent pour lisser les rides et ridules et/ou retendre la peau et le document FR 2 822 676 décrit une composition cosmétique filmogène comprenant au moins, à titre d'agent filmogène, un copolymère acrylique dans une forte teneur, à savoir comprise entre 20 et 50 % en poids par rapport au poids de la composition.

Par ailleurs, il est connu que les émulsions sont d'un usage fréquent en cosmétique. Celles-ci offrent en effet une grande flexibilité dans les formulations et dans les domaines d'applications cosmétiques. Elles peuvent ainsi en particulier inclure une grande variété d'ingrédients actifs de nature très différente et se présenter sous diverses formes telles que des crèmes ou des gels plus ou moins fluides ou épais.

Les émulsions simples sont essentiellement constituées de deux phases non miscibles, l'une grasse et l'autre aqueuse, et d'un tensio-actif dont le rôle est de stabiliser la dispersion de l'une des phases, qui se trouve présente sous la forme de gouttelettes dispersées, au sein de la phase continue. Selon la proportion des deux phases au sein de l'émulsion et de la nature du tensio-actif, celle-ci est dite du type huile-dans-eau (H/E), lorsque la phase aqueuse est la phase continue (émulsions directes), ou dite du type eau-dans-huile (E/H), lorsque la phase grasse est la phase continue (émulsions inverses).

Malheureusement, certains agents tenseurs de type polymères synthétiques présentent l'inconvénient majeur de conférer aux compositions sous formes d'émulsion dans lesquelles ils sont formulés une stabilité insuffisante. Cette instabilité peut apparaître au bout de quelques jours, voire quelques semaines. Elle peut se traduire par une diminution très importante de la viscosité au cours du temps de stockage, et/ou par un aspect « caillé », voire déphasé de l'émulsion.

Une telle instabilité rend difficile l'utilisation de ces copolymères tenseurs de type polymères synthétiques dans des compositions cosmétiques sous forme d'émulsion, ces dernières devant présenter une excellente stabilité pendant toute leur durée de vie commerciale.

Il existe donc un besoin de formuler des compositions cosmétiques sous forme d'émulsion stable comprenant un agent tenseur de type polymère synthétique offrant un effet tenseur intéressant.

Les inventeurs ont découvert de façon inattendue que les copolymères statistiques à chaîne principale linéaire de nature éthylénique, caractérisés en ce qu'ils :

(i) présentent une masse moléculaire en poids comprise entre 15 000 et 600 000 g/mol,

(ii) contiennent au moins 70 % d'« unités monomères » issues de monomères dont les homopolymères sont hydrophobes et présentent une température de transition vitreuse supérieure à 40 °C,

(iii) contiennent également au moins une « unité monomère » issue d'un monomère hydrophile ionique au moins partiellement neutralisée, et

(iv) présentent une température globale de transition vitreuse supérieure ou égale à 45 °C,

présentaient des effets tenseurs très intéressants tout en pouvant être formulés sous forme d'émulsion stable. Ces composés présentent ainsi une rigidité très importante après application sur la peau et évaporation des matières volatiles tout en offrant un effet tenseur immédiat, suffisant et durable, sans risque pour le consommateur. Ces polymères permettent également de réaliser des émulsions stables dans le temps.

La présente invention a donc pour objet une composition cosmétique comprenant, dans un milieu physiologiquement acceptable, au moins un copolymère statistique à chaîne principale linéaire de nature éthylénique, dans laquelle ledit copolymère :

(i) présente une masse moléculaire en poids comprise entre 15 000 et 600 000 g/mol,

(ii) contient au moins 70 % d'« unités monomères » issues de monomères dont les homopolymères sont hydrophobes et présentent une température de transition vitreuse supérieure à 40 °C,

(iii) contient également au moins une « unité monomère » issue d'un monomère hydrophile ionique au moins partiellement neutralisée, et

(iv) présente une température globale de transition vitreuse supérieure ou égale à 45 °C,

et se présentant sous la forme d'une émulsion eau-dans-huile ou d'une émulsion multiple.

L'invention a également pour objet l'utilisation comme agent tenseur, dans une composition cosmétique sous forme d'émulsion eau-dans-huile ou d'émulsion multiple, d'au moins un copolymère statistique à chaîne principale linéaire de nature éthylénique, ledit copolymère étant tel que défini précédemment.

Dans le cadre de cette description et des revendications annexées, on entend par « agent tenseur » des composés susceptibles d'avoir un effet tenseur apparent, c'est-à-dire de lisser la peau et réduire, voire faire disparaître, de façon immédiate les rides et les ridules.

La composition sous forme d'émulsion eau-dans-huile ou d'émulsion multiple utilisée dans la présente invention contient, en plus du copolymère précité, un milieu physiologiquement acceptable, c'est-à-dire compatible avec la peau et ses phanères, les muqueuses et les semi-muqueuses.

La présente invention se rapporte également à un procédé de traitement cosmétique d'une peau vieillie, notamment ridée, comprenant l'application sur ladite peau d'au moins une composition selon l'invention, en une quantité efficace pour estomper les rides par un effet tenseur.

Le terme « de nature éthylénique » dans la cadre de la présente invention qualifie les polymères comportant une chaîne principale comportant uniquement des unités monomères,

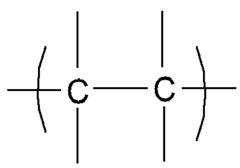

les chaînes latérales pouvant être constituées d'atomes de carbone, d'oxygène, d'azote, d'hydrogène, de soufre et/ou de phosphore.

On note en particulier que la présence d'atomes de silicium dans la chaîne latérale est exclue de la portée de l'invention.

Le terme « unité monomère » désigne l'unité constitutive la plus grande de la structure d'une macromolécule formée à partir d'une même molécule de monomère.

Le terme « alkyle », dans le cadre de la présente invention, signifie une chaîne hydrocarbonée linéaire ou ramifiée, saturée ou insaturée, cyclique ou non cyclique. Parmi les groupes alkyle convenant à la mise en oeuvre de l'invention, on peut notamment citer le groupe méthyle, éthyle, isopropyle, n-propyle, n-butyle, t-butyle, - $CH_2$-t-butyle, pentyle, n-hexyle, cyclopropyle, cyclopentyle, cyclohexyle, cyclohexylméthyle, heptyle, octyle, nonyle, décyle, norbornyle et adamantyle.

Le terme « aryle », dans le cadre de la présente invention, signifie un système mono- ou bicyclique possédant

un ou deux noyaux aromatiques. Parmi les groupes aryle, on peut citer le phényle, le napthyle, le tétrahydronaphtyle, l'indanyle.

Le terme « aralkyle » signifie un groupe aryle lié à un groupe alkyle, tel que le groupe benzyle.

Le terme « groupe hétérocyclique » signifie un cycle de 4 à 12 chaînons contenant un ou plusieurs hétéroatomes identiques ou non choisis parmi O, N, S ou P. Ledit groupe hétérocyclique peut comprendre ou non des doubles liaisons. Ce terme comprend en outre les groupes bicycliques dans lesquels un hétérocycle à 3, 4, 5 ou 6 chaînons est fusionné avec un groupe phényle ou avec un cycloalkyle tel que le cyclohexane ou encore avec un autre hétérocycle.

Parmi ces groupes hétérocycliques, on peut citer l'indolyle, le quinolyle, l'isoquinolyle, le tétrahydroquinolyl, le benzofuryle et le benzothiènyle. Le terme « groupe hétérocyclique » recouvre notamment le pyrrolyle, le pyrrolinyle, le pyrrolidinyle, le pyrazolyle, le pyrazolinyle, le pyrazolidinyle, l'imidazolyle, l'imidazolinyle, l'imidazolidinyle, le pyridyle, le pipéridinyle, le pyrazinyle, le pipérazinyle, le pyrimidinyle, le pyridazinyle, l'oxazolyle, l'oxazolidinyle, l'isoxazolyle, l'isoxazolidinyle, le morpholinyle, le thiazolyle, le thiazolidinyle, l'isothiazolyle, l'isothiazolidinyle, l'indolyle, le quinolinyle, l'isoquinolinyle, le benzimidazolyle, le benzothiazolyle, le benzoxazolyle, le furyle et le thiényle.

Le terme « hétérocyclolakyle » signifie un groupe hétérocyclique lié à un groupe alkyle.

Les atomes d'halogène comprennent le chlore, le brome, l'iode et le fluor.

Les hétéroatomes, sauf mention contraire, comprennent les atomes d'oxygène, d'azote, de soufre et de phosphore.

Par « émulsion », on entend, au sens de la présente invention, un système à au moins deux phases constitué de deux liquides ou plus, non miscibles ou partiellement miscibles entre eux, dont l'un - qui forme la phase dispersée - est dispersé dans l'autre - qui forme la phase continue - sous forme de fines gouttelettes, dont le diamètre n'excède pas 5 microns.

Le terme « composition stable » désigne une composition qui se conserve bien dans le temps, notamment après 1 mois, voire 2 mois, à température ambiante (25 °C), sans déphasage ni décantation.

Le terme « composition instable » désigne une composition qui présente une décantation ou un déphasage après conservation d'au moins 1 mois à température ambiante.

La stabilité des émulsions eau-dans-huile ou multiples se traduit notamment par une bonne dispersion de la phase aqueuse dans la phase grasse et l'absence d'amas de gouttes aqueuses, favorisant l'apparition de domaines d'eau de diamètre important. Le comportement de l'émulsion peut être mis en évidence par un simple test de dilution de l'émulsion par de l'huile, suivi de son observation au microscope. Dans le cas d'une émulsion instable, les gouttes sont agrégées et forment des amas, ce qui favorise la sédimentation et la formation de domaines d'eau ; l'émulsion est alors considérée comme instable du fait de son hétérogénéité créée par la présence de ses domaines d'eau. Dans le cas contraire, on observe une bonne dispersion des gouttes de phase aqueuse et une bonne stabilité de l'émulsion qui est homogène.

Les termes « compris entre ... et... » et « allant de... à » signifient que les bornes sont également décrites.

## COPOLYMERES

Le copolymère présent dans la composition selon la présente invention est un copolymère statistique à chaîne principale linéaire de nature éthylénique.

Il peut en particulier s'agir d'un copolymère statistique à chaîne principale linéaire de nature éthylénique :

(i) présentant une masse moléculaire en poids comprise entre 15 000 et 600 000 g /mol, et consistant en :
(ii) au moins 70 % en poids d'« unités monomères » issues de monomères dont les homopolymères sont hydrophobes et présentent une température de transition vitreuse supérieure à 40 °C,
(iii) au moins une « unité monomère » issue d'un monomère hydrophile ionique au moins partiellement neutralisée, et
(iv) 0 à 25 % en poids d'« unités monomères » issues de monomères additionnels dont les homopolymères présentent une température de transition vitreuse inférieure à 40 °C choisis parmi :

- les acrylates de formule :

$$CH_2 = CHCOOR_{12}$$

où $R_{12}$ représente un groupe alkyle en $C_1$ à $C_{12}$, linéaire ou ramifié (à l'exception du groupe tertiobutyle) dans lequel se trouve(nt) éventuellement intercalé(s) un ou plusieurs hétéroatomes identiques ou non, ledit groupe alkyle pouvant en outre être éventuellement substitué par un ou plusieurs substituants identiques ou non choisis parmi les groupes hydroxyle et les atomes d'halogène, et

- les méthacrylates de formule :

$$CH_2 = C(CH_3)COOR_{14}$$

où $R_{14}$ représente un groupe alkyle en $C_4$ à $C_{12}$ linéaire ou un groupe alkyle en $C_5$ à $C_{12}$ ramifié, et
(v) présente une température globale de transition vitreuse supérieure ou égale à 45 °C.

La composition peut également comprendre un mélange de tels copolymères.
Le copolymère utile dans le cadre de la présente invention présente une masse moléculaire en poids comprise ente 15 000 et 600 000 g/mol. La masse moléculaire est de préférence comprise entre 20 000 et 200 000 g/mol, et va de façon encore plus préférée de 55 000 à 200 000 g/mol. Par « masse moléculaire en poids », on entend la masse moléculaire Mp au pic de la courbe de distribution.
Par ailleurs, sa température globale de transition vitreuse est supérieure ou égale à 45 °C, notamment allant de 45 °C à 300 °C.
On entend par « température de transition vitreuse », dont l'abréviation est « Tg », la température au-dessous de laquelle le polymère est rigide. Lorsque la température augmente, le polymère passe par un état de transition qui permet aux chaînes macromoléculaires de glisser les unes par rapport aux autres et le polymère se ramollit.
On utilise le terme « température globale de transition vitreuse » pour signifier que le copolymère peut comporter des monomères distincts, dont les homopolymères respectifs peuvent présenter des températures de transition vitreuse différentes et que c'est le copolymère en tant que tel qui présente ladite température globale de transition vitreuse.
Ainsi, on préfère dans le cadre de la présente invention utiliser des copolymères présentant une température globale de transition vitreuse supérieure à 60 °C, notamment supérieure à 60 °C et inférieure à 300 °C.
Dans le cadre de la présente invention, le protocole de mesure de la température de transition vitreuse des copolymères ou des homopolymères formés par les monomères utiles pour la préparation des copolymères utilise une caractérisation par DSC (Differential Scanning Calorimetry) et est détaillé ci-après :
Les transitions du film (transitions vitreuses, fusions...) sont étudiées par DSC sur la base de 2 cycles de chauffage / refroidissement à 10 °C/min entre -140 °C et 130 °C (environ 2 heures). Les mesures sont réalisées sous balayage d'azote et en utilisant des coupelles hermétiques pour ne pas modifier la composition du film, par vaporisation du solvant, au cours de l'étude DSC. Le film de polymère est préparé par séchage de la solution aqueuse directement déposée (40 μl) dans les creusets d'analyse thermique. Le séchage de la solution se fait dans des conditions contrôlées pendant 48 heures à température ambiante et à 50 +/- 5 % d'humidité relative.

- Appareil : DSC 2920 de TA Instruments
- Gaz de purge : Azote Alphagaz 2 à 50 ml/min
- Creuset : coupelle Inox sertie de 50 μl de Perkin Elmer
- Calibration en énergie et température : Fusion de l'Indium
- Prise d'essai : conditionnée par le séchage (environ 10 mg)
- Traitements thermiques :

    1. CO : Refroidissement de +25 °C à - 140 °C à 10 °C/min
    2. CO bis : Equilibrage à - 140 °C
    3. H1: Chauffage de - 140 °C à + 130 °C à 10 °C/min
    4. C1 : Refroidissement de + 130 °C à - 140 °C à 10 °C/min
    5. C1 bis : Equilibrage à - 140 °C
    6. H2 : Chauffage de - 140 °C à + 130 °C à 10 °C/min

Deux échantillons sont étudiés pour chaque produit.
Le copolymère selon l'invention peut se présenter sous forme d'une dispersion aqueuse.
La structure du copolymère est détaillée dans la description qui va suivre.
Le copolymère comprend au moins 70 % en poids par rapport à son poids total d'« unités monomères » issues de monomères dont les homopolymères présentent une température de transition vitreuse supérieure à 40 °C. Ces « unités monomères » peuvent être de la même nature ou de nature différente. Autrement dit, le copolymère peut comprendre un seul et même type d'« unités monomères » dont l'homopolymère présente une température de transition vitreuse supérieure à 40 °C ou bien des « unités monomères » de natures distinctes, étant entendu que les monomères correspondants sont tous conformes à l'exigence rappelée ci-dessus concernant la température de transition vitreuse.
En particulier, le copolymère selon l'invention comprend plus de 72 %, notamment plus de 75 %, de préférence plus de 80 % en poids par rapport au poids total, d'« unités monomères » issues de monomères dont les homopolymères

présentent une température de transition vitreuse supérieure à 40 °C, et notamment jusqu'à 95 % en poids.

Le copolymère comprend en outre au moins une « unité monomère » issue d'un monomère hydrophile ionique, ainsi que de 0 à 25 % en poids d'« unités monomères » issues de monomères additionnels dont les homopolymères présentent une température de transition vitreuse inférieure à 40 °C.

*Monomères hydrophobes dont les homopolymères présentent une température de transition vitreuse supérieure à 40 °C*

Par « monomère hydrophobe », on entend, au sens de la présente invention, un monomère dont l'homopolymère est insoluble dans l'eau à une concentration supérieure à 5 % en poids, à 25 °C, et qui ne forme pas non plus dans l'eau, dans ces conditions, une dispersion ou suspension stable de fines particules, généralement sphériques, ayant une taille moyenne de particule inférieure à 1 µm, et plus généralement comprise entre 5 et 400 nm, voire entre 10 et 250 nm, telle que mesurée par diffusion de la lumière.

Parmi les monomères dont sont issues les « unités monomères » hydrophobes citées précédemment, on préfère ceux dont les homopolymères présentent une température de transition vitreuse supérieure ou égale à 60 °C, et notamment inférieure ou égale à 300 °C.

Les monomères utiles pour la préparation des copolymères compris dans les compositions selon la présente invention et dont les homopolymères ont des températures de transition vitreuse supérieures à 40°C sont, de préférence, choisis parmi les composés vinyliques, les acrylates, les métacrylates et les (meth)acrylamides, et en particulier parmi les monomères suivants :

- les composés vinyliques de formule :

$$CH_2 = CHR_1,$$

où $R_1$ est un groupe

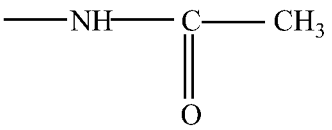 , un groupe 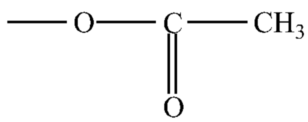 ;

un groupe cycloalkyle en $C_3$ à $C_8$ un groupe aryle en $C_6$ à $C_{20}$ ; un groupe aralkyle en $C_7$ à $C_{30}$ (groupe alkyle en $C_1$ à $C_4$) ; un groupe hétérocyclique ; un groupe hétérocyclylalkyle (alkyle en $C_1$ à $C_4$) tel qu'un groupe furfuryle ; lesdits groupes cycloalkyle, aryle, aralkyle, hétérocyclique, ou hétérocyclylalkyle pouvant être éventuellement substitués par un ou plusieurs substituants identiques ou non choisis parmi les groupes hydroxyle, les atomes d'halogène, et les groupes alkyles en $C_1$ à $C_4$ linéaires ou ramifiés dans lesquels se trouve(nt) éventuellement intercalé(s) un ou plusieurs hétéroatomes identiques ou non, et lesdits groupes alkyle pouvant, en outre, être éventuellement substitués par un ou plusieurs substituants choisis parmi les groupes hydroxyle et les atomes d'halogène.

Selon une variante préférée, $R_1$ est un groupe

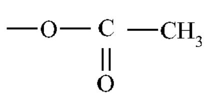 ,

un groupe cycloalkyle en $C_3$ à $C_8$ ou un groupe aryle en $C_6$ à $C_{20}$.

Des exemples particulièrement préférés de monomères vinyliques dont les homopolymères présentent une température de transition vitreuse supérieure à 40 °C sont le vinylcyclohexane, le styrène et l'acétate de vinyle.

- les acrylates de formule

$$CH_2 = CHCOOR_2$$

où $R_2$ est un groupe tertiobutyle ; un groupe cycloalkyle en $C_3$ à $C_8$ éventuellement ponté par un groupe alkylène en $C_1$ à $C_4$ éventuellement substitué par un plusieurs groupes alkyles en $C_1$ à $C_4$ ; un groupe aryle en $C_6$ à $C_{20}$ ; un groupe aralkyle en $C_7$ à $C_{30}$ (groupe alkyle en $C_1$ à $C_4$) ; un groupe hétérocyclique ; un groupe hétérocyclylalkyle (alkyle en $C_1$ à $C_4$); lesdits groupes cycloalkyle, aryle, aralkyle, hétérocyclique ou hétérocyclylalkyle pouvant être éventuellement substitués par un ou plusieurs substituants identiques ou non choisis parmi les groupes hydroxyle, les atomes d'halogène, et les groupes alkyle en $C_1$ à $C_4$ linéaires ou ramifié dans lesquels se trouve(nt) éventuellement intercalé(s) un ou plusieurs hétéroatomes identiques ou non, lesdits groupes alkyle pouvant, en outre, être éventuellement substitués par un ou plusieurs substituants identiques ou non choisis parmi les groupes hydroxyle et les atomes d'halogène.

Selon une variante préférée, $R_2$ est un groupe tertiobutyle, un groupe cycloalkyle en $C_3$ à $C_8$ éventuellement ponté par un groupe alkylène en $C_1$ à $C_4$, ou un groupe aralkyle en $C_7$ à $C_{30}$ (groupe alkyle en $C_1$ à $C_4$).

Des exemples particulièrement préférés d'acrylates dont les homopolymères présentent une température de transition vitreuse supérieure à 40°C sont l'acrylate de benzyle, l'acrylate de cyclohexyle, l'acrylate de tertiobutyle, l'acrylate d'isobornyle et l'acrylate de norbornyle.

- les méthacrylates de formule

$$CH_2 = C(CH_3)\ COOR_3$$

où $R_3$ est un groupe isobutyle ou tertiobutyle ou un groupe alkyle en $C_1$ à $C_3$, linéaire ou ramifié, tel qu'un groupe méthyle, éthyle, propyle ou isobutyle, ces groupes alkyle pouvant en outre être éventuellement substitués par un ou plusieurs substituants identiques ou non choisis parmi les groupes hydroxyle et les atomes d'halogène ; un groupe cycloalkyle en $C_3$ à $C_8$ éventuellement ponté par un groupe alkylène en $C_1$ à $C_4$ éventuellement substitué par un plusieurs groupes alkyle en $C_1$ à $C_4$ ; un groupe aryle en $C_6$ à $C_{20}$ ; un groupe aralkyle en $C_7$ à $C_{30}$ (groupe alkyle en $C_1$ à $C_4$) ; un groupe hétérocyclique ; un groupe hétérocyclylalkyle (alkyle en $C_1$ à $C_4$) ; lesdits groupes cycloalkyle, aryle, aralkyle, ou hétérocyclique ou hétérocyclylalkyle pouvant être éventuellement substitués par un ou plusieurs substituants identiques ou non choisis parmi les groupes hydroxyle, les atomes d'halogène, et les groupes alkyle en $C_1$ à $C_4$ linéaires ou ramifié dans lequel se trouve(nt) éventuellement intercalé(s) un ou plusieurs hétéroatomes identiques ou non, lesdits groupes alkyle pouvant, en outre, être éventuellement substitués par un ou plusieurs substituants identiques ou non choisis parmi les groupes hydroxyle et les atomes d'halogène.

Selon une variante préférée, $R_3$ est un groupe isobutyle, tertiobutyle, un groupe alkyle en $C_1$ à $C_3$ linéaire ou ramifié, un groupe cycloalkyle en $C_3$ à $C_8$ éventuellement ponté par un groupe alkylène en $C_1$ à $C_4$ ou un groupe aralkyle en $C_7$ à $C_{30}$ (groupe alkyle en $C_1$ à $C_4$).

Des exemples particulièrement préférés de méthacrylates dont les homopolymères présentent une température de transition vitreuse supérieure à 40°C sont le méthacrylate de méthyle, d'éthyle, d'isobutyle, de cyclohexyle, de benzyle, de tertiobutyle, d'isobornyle et de norbornyle.

- les (méth)acrylamides de formule :

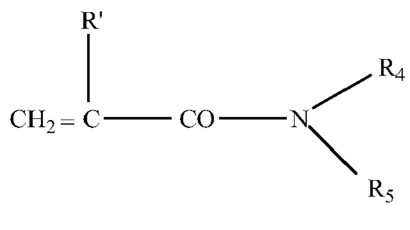

où R' désigne H ou $-CH_3$, et où $R_4$ et $R_5$ identiques ou différents représentent chacun un atome d'hydrogène ou un groupe alkyle linéaire ou ramifié en $C_4$ à $C_{12}$, étant entendu que $R_4$ et $R_5$ ne peuvent représenter simultanément un atome d'hydrogène.

Des exemples particulièrement préférés de monomères (méth)acrylamide dont les homopolymères présentent une température de transition vitreuse supérieure à 40°C sont le N-butylacrylamide, le N-t-butylacrylamide et le N,N-dibutylacrylamide.

Selon un mode de réalisation préféré de l'invention, les monomères dont les homopolymères présentent une température de transition vitreuse supérieure à 40°C sont choisis parmi le styrène, l'acrylate de benzyle, l'acrylate de

cycloalkyle en $C_3$ à $C_8$ éventuellement ponté par un groupe alkylène en $C_1$ à $C_4$, l'acrylate de tertiobutyle, le méthacrylate de ($C_1$-$C_3$)alkyle, le méthacrylate de tertiobutyle, le méthacrylate de benzyle et le méthacrylate de cycloalkyle en $C_3$ à $C_8$ éventuellement ponté par un groupe alkylène en $C_1$ à $C_4$.

Les monomères dont les homopolymères présentent une température de transition vitreuse supérieure à 40°C préférés sont l'acrylate de benzyle, l'acrylate de cyclohexyle, l'acrylate de tertiobutyle, l'acrylate d'isobornyle et l'acrylate de norbornyle, le méthacrylate de méthyle, d'éthyle, d'isobutyle, de cyclohexyle, de benzyle, de tertiobutyle, d'isobornyle et de norbornyle et le styrène. Plus préférentiellement, ils sont choisis parmi les méthacrylates de méthyle et le méthacrylate de cyclohexyle.

*Monomère hydrophile ionique*

Le copolymère présent dans la composition selon la présente invention comprend en outre au moins une « unité monomère » issue d'un monomère hydrophile ionique au moins partiellement neutralisée.

Ce monomère hydrophile ionique peut notamment être choisi parmi les monomères hydrophiles anioniques, les monomères hydrophiles cationiques, les monomères amphotères et leurs mélanges.

Typiquement, le copolymère peut contenir entre 5 et 30 % en poids d'« unités monomères » issues de monomères hydrophiles ioniques par rapport à son poids total. Plus particulièrement, le copolymère peut contenir entre 5 et 25 % en poids, notamment entre 5 et 20 % en poids, de préférence entre 5 et 18 % en poids d'« unités monomères » issues de monomères hydrophiles ioniques par rapport à son poids total. Le copolymère peut par exemple contenir entre 10 et 25 % en poids, notamment entre 10 et 20 % en poids, de préférence entre 10 et 18 % en poids d'« unités monomères » issues de monomères hydrophiles ioniques par rapport à son poids total.

Selon un mode préféré de l'invention, le copolymère contient entre 5 et 25 % en poids d'« unités monomères » issues de monomères hydrophiles ioniques, étant entendu que lorsque le copolymère contient un ou des monomère(s) additionnel(s) dont le ou les homopolymère(s) (respectifs) présente(nt) une température de transition vitreuse inférieure à 40 °C, l'un au moins de ces monomère(s) additionnel(s) étant l'acrylate d'étylhexyle, le copolymère contient alors entre 5 et 18 % en poids d'« unités monomères » issues de monomères hydrophiles ioniques.

Par « partiellement neutralisé », on entend au sens de la présente invention, un taux de neutralisation non nul, et notamment supérieur ou égal à 50 %.

Avantageusement, l'ensemble des « unités monomères » présentent un taux de neutralisation supérieur ou égal à 50 %, de préférence supérieur ou égal à 70 %, en particulier d'au moins 90 %, voire de 100 %.

Le taux de neutralisation peut être défini comme étant le rapport du nombre de fonctions ioniques neutralisées sur le nombre de fonctions ionisées initiales, c'est-à-dire avant neutralisation.

De façon avantageuse, le taux de neutralisation de l'ensemble des « unités monomères » sera ajusté de sorte à permettre une mise en dispersion dans l'eau des copolymères selon l'invention.

Parmi les monomères hydrophiles anioniques, sont compris les monomères de formule (I) :

$$CH_2{=}CR_6\,(Z)_n\,(R_7)_m\,Y \qquad (I)$$

où Z prend l'une des significations suivantes : C(=O)O, C(=O)NH, O, O(C=O) (acétates),
n est égal à 0 ou 1, de préférence égal à 0,
m est égal à 0 ou 1, de préférence égal à 0,
$R_6$ est un atome d'hydrogène, un groupe $CH_3$, ou un groupe ($C_2$-$C_{30}$)alkyle,
$R_7$ est choisi parmi les groupements alkylènes linéaires saturés ou non et/ou branchés et/ou cycliques (aromatiques ou non) en $C_1$ à $C_{30}$, incluant ou non un ou plusieurs hétéroatomes, et
Y est choisi parmi les groupes suivants : -COOH, $-SO_3H$, $-OSO_3H$, $OP(OH)_2$, $-OPO(OH)_2$

Préférentiellement $R_6$ est un atome d'hydrogène, un groupe $CH_3$ ou $C_2H_5$, de préférence $CH_3$.

$R_7$ est par exemple choisi parmi les groupes alkyle en $C_1$ à $C_{30}$, phénylène, benzylène, $-(CH_2-CH=CH)-$ et -(CHOH)-. De préférence $R_7$ est choisi parmi les groupes alkylène en $C_1$ à $C_6$ linéaires ramifiés ou cycliques, les groupes phénylène et benzylène.

Selon un mode de réalisation particulier, $R_6$ est un atome d'hydrogène, un groupe $CH_3$ ou un $C_2H_5$, de préférence un groupe $CH_3$ et $R_7$ est choisi parmi les groupes alkylène en $C_1$ à $C_{30}$, phénylène, benzylène, $(CH_2-CH=CH)-$ et -(CHOH).

On peut citer notamment parmi les monomères hydrophiles anioniques :

- les monomères à insaturation(s) éthylénique(s) comprenant au moins une fonction acide carboxylique (COOH), phosphonique ($PO_3H_2$) ou sulfonique ($SO_3H$), comme par exemple l'acide acrylique, l'acide méthacrylique, le (méth) acrylate de 2-carboxyéthyle, l'acide vinylbenzoïque, l'acide acrylamidoglycolique $CH_2{=}CHCONHCH(OH)CO_2H$, l'acide maléique, l'acide acrylamidopropanesulfonique, l'acide acrylamido 2-méthylpropanesulfonique, l'acide styrène sulfonique, l'acide vinylsulfonique, le méthacrylate ou l'acrylate d'acide propyl-3-sulfonique ($CH_2{=}C(CH_3)CO_2$

$(CH_2)_3SO_3H$), le méthacrylate ou l'acrylate d'acide éthyl-2-sulfonique ($CH_2=C(CH_3)CO_2(CH_2)_2SO_3H$) et la vinylméthylsulfone, l'acide vinylphosphonique ($CH_2=CH-PO(OH)_2$), le méthacrylate d'acide éthyl-2-phosphonique ($CH_2=C(CH_3)COOCH_2CH_2OP(O)(OH)_2$),

- les anhydrides carboxyliques porteurs d'une liaison vinylique tels que l'anhydride maléique,
- les diacides tels que l'acide crotonique, l'acide itaconique, l'acide fumarique, l'acide maléique,
- ainsi que leurs mélanges.

De préférence, le monomère hydrophile anionique est l'acide (méth)acrylique.

La neutralisation des groupes anioniques peut être effectuée par une base minérale, telle que LiOH, NaOH, KOH, $Ca(OH)_2$, $NH_4OH$ ou $Zn(OH)_2$; ou par une base organique telle qu'une alkylamine primaire, secondaire ou tertiaire, notamment la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine et la diméthylamino-2-propanol. On peut encore citer la lysine ou la 3-(diméthylamino)propylamine.

Parmi les monomères hydrophiles cationiques, sont compris les monomères de formule (II) :

$$CH_2=CR_8\ (Z)_n\ (R_9)_m\ X \qquad (II)$$

où

- Z prend l'une des significations suivantes : C(=O)O, C(=O)NH, O, O(C=O),
- n est égal à 0 ou 1,
- m est égal à 0 ou 1,
- $R_8$ est un atome d'hydrogène, un groupe $CH_3$, ou un groupe ($C_2$ à $C_{30}$)alkyle,
- $R_9$ est choisi parmi les groupements alkylènes linéaires saturés ou non et/ou branchés et/ou cycliques, aromatiques ou non, en $C_1$ à $C_{30}$, incluant ou non un ou plusieurs hétéroatomes,
- X est un groupe défini par $N-R_{10}R_{11}$, ou bien X constitue un cycle, aromatique ou non, comportant un groupement amine tertiaire cationisable, inclus dans le cyle ou comme substituant, ou peut représenter un hétérocycle aromatique, ou non, contenant un azote tertiaire, cationisable inclus dans le cycle ou comme substituant ou bien X prend l'une des significations suivantes : guanidino, amidino, ou phosphino, et
- $R_{10}$ et $R_{11}$ sont choisis indépendamment parmi un atome d'hydrogène et des groupements alkyles linéaires et/ou branchés et/ou cycliques,aromatiques ou non, en $C_1$ à $C_{16}$, comportant ou non des hétéroatomes ou bien $R_{10}$ et $R_{11}$ forment avec l'atome d'azote auquel ils sont rattachés, un hétérocycle en $C_4$ à $C_{30}$, fusionné ou non, et éventuellement substitué par un ou plusieurs radicaux identiques ou non choisis parmi les groupes alkyle en $C_1$ à $C_4$, le groupe hydroxyle, un groupe alcoxy en $C_1$ à $C_4$, et un atome d'halogène.

$R_9$ est de préférence choisi parmi les groupes alkyle en $C_1$ à $C_{30}$, phénylène, benzylène, $-(CH_2-CH=CH)-$ et -(CHOH)-.

Avantageusement, $R_9$ est choisi parmi les groupes alkylène en $C_1$ à $C_6$ linéaires ramifiés ou cycliques, les groupes phénylènes et benzylènes.

De préférence $R_{10}$, $R_{11}$ sont choisis parmi un atome d'hydrogène et les groupes $CH_3$, $C_2H_5$, $C_3H_7$ et $C_4H_9$.

Des exemples d'hétérocycles convenant à la signification de X dans sa deuxième alternative, sont des pyridines, indolyle, isoindolinyle, imidazolyle, imidazolinyle, pipéridinyle, pyrazolynyle, pyrazolyle, quinoline, pyrazolinyle, pyridinyle, pipérazinyle, pyrrolidinyle, quinidinyle, thiazolinyle, morpholine et leurs mélanges.

Comme exemples de monomères hydrophiles cationiques, on peut citer les monomères à insaturation(s) éthylénique(s) comprenant au moins une fonction amine tertiaire ou secondaire ou primaire. On peut notamment citer :

- l'allylamine, allylpyridine ;
- les (méth)acrylates d'aminoalkyles, tels que les (méth)acrylates de [N,N-di($C_1$-$C_4$)alkylamino]($C_1$-$C_6$)alkyle ou les (méth)acrylates de [N-($C_1$-$C_4$)alkylamino]($C_1$-$C_6$)alkyle et notamment le (méth)acrylate de N,N diméthylaminoéthyle, le (méth)acrylate de N,N-diéthylaminoéthyle, le (méth)acrylate de 2-aminoéthyle, le (méth)acrylate de 2-(N-terbutylamino)éthyle;
- les (méth)acrylamides d'aminoalkyles, tels que les (méth)acrylamides de [N,N-di($C_1$-$C_4$)alkylamino]($C_1$-$C_6$)alkyle N,N-di($C_1$-$C_4$)alkylamino)alkyl ou les (méth)acrylamides [N-($C_1$-$C_4$)alkylamino]($C_1$-$C_6$)alkyle, et notamment le (méth)acrylamide de N,N-diméthylaminopropyle, le (méth)acrylamide de N,N-diméthylaminoéthyle et le (méth) acrylamide de 3-aminopropyle;
- la vinylamine, le 2-(diéthylamino)éthylstyrène;
- N-vinylimidazole, N-vinyl-2-méthylimidazole, N-vinylcarbazole ;

- ainsi que leurs mélanges, ou leurs formes quaternisées.

Pour neutraliser les monomères cationiques, on peut utiliser les sels d'acides minéraux, tels que l'acide sulfurique, l'acide chlorhydrique, l'acide bromhydrique, l'acide iodhydrique, l'acide phosphorique et l'acide borique.
On peut également citer les sels d'acides organiques, qui peuvent comporter un ou plusieurs groupes acide carboxylique, sulfonique ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.
Les groupes amines tertiaires peuvent être quaternisés :

- par des composés à halogène mobile, notamment des halogénures d'alkyles tels que des chlorures ou des bromures d'alkyles en $C_1$ à $C_{12}$, et par exemple le bromure de méthyle ou le chlorure d'éthyle,
- par des composés à halogène mobile, comportant des fonctions acides carboxyliques ou sulfoniques (éventuellement salifiées). Ainsi sont obtenus des monomères hydrophiles amphotères (encore dénommés bétaines).

Les quaternisants peuvent par exemple être le chloroacétate de sodium ou des sulfones cycliques, par exemple la propanesulfone.
Cette réaction de quaternisation et (ou) de salification peut avoir lieu sur le polymère déjà synthétisé ou sur le monomère de départ avant de procéder à la polymérisation.
Comme exemples de monomères amphotères, on peut citer les carboxybétaïnes ou sulfobétaïnes éthyléniques telles que la N,N-diméthyl-N-(2-méthacryloyloxyéthyl)-N-(3-sulfopropyl) ammonium bétaine ; la N,N-diméthyl-N-(3-méthacrylamidopropyl)-N-(3-sulfopropyl)ammonium bétaine et la 1-(3-sulfopropyl)-2-vinylpyridinium bétaine.
Le monomère hydrophile cationique préféré est le N,N' diméthylaminoéthyl (méth)acrylate.
Le monomère hydrophile préféré est l'acide (méth)acrylique.

*Copolymères préférés*

Selon un mode de réalisation particulièrement préféré, le copolymère est choisi parmi

- Les copolymères de méthacrylate de méthyle / acide méthacrylique ; les copolymères de méthacrylate de méthyle / acide acrylique, lesdits copolymères contenant entre 70 et 90 % en poids de méthacrylate de méthyle ;
- Les copolymères de méthacrylate d'éthyle/ acide méthacrylique ; les copolymères de méthacrylate d'éthyle / acide acrylique, lesdits copolymères contenant entre 70 et 90 % en poids de méthacrylate d'éthyle ;
- Les copolymères de méthacrylate d'isobutyle/ acide méthacrylique ; les copolymères de méthacrylate d'isobutyle / acide acrylique, lesdits copolymères contenant entre 70 et 90 % en poids de méthacrylate d'isobutyle ;
- Les copolymères de méthacrylate de benzyle / acide méthacrylique ; les copolymères de méthacrylate de benzyle / acide acrylique, lesdits copolymères contenant entre 70 et 90 % en poids de méthacrylate de benzyle ;
- Les copolymères d'acrylate de benzyle / acide méthacrylique ; les copolymères d'acrylate de benzyle / acide acrylique, lesdits copolymères contenant entre 70 et 90 % en poids d'acrylate de benzyle ;
- Les copolymères de méthacrylate de cyclohexyle / acide méthacrylique ; les copolymères de méthacrylate de cyclohexyle / acide acrylique, lesdits copolymères contenant entre 70 et 90 % en poids de méthacrylate de cyclohexyle ;
- Les copolymères d'acrylate de cyclohexyle / acide méthacrylique ; les copolymères d'acrylate de cyclohexyle / acide acrylique, lesdits copolymères contenant entre 70 et 90 % en poids d'acrylate de cyclohexyle ;
- Les copolymères de méthacrylate de tertio-butyle/ acide méthacrylique ; les copolymères de méthacrylate de tertio-butyle / acide acrylique, lesdits copolymères contenant entre 70 et 90 % en poids de méthacrylate de tertiobutyle ;
- Les copolymères d'acrylate de tertio-butyle/ acide méthacrylique ; les copolymères d'acrylate de tertio-butyle / acide acrylique, lesdits copolymères contenant entre 70 et 90 % en poids d'acrylate de tertio-butyle ;
- Les copolymères de méthacrylate d'isobornyle/ acide méthacrylique ; les copolymères de méthacrylate d'isobornyle / acide acrylique, lesdits copolymères contenant entre 70 et 90 % en poids de méthacrylate d'isobornyle ;
- Les copolymères d'acrylate d'isobornyle / acide méthacrylique ; les copolymères d'acrylate d'isobornyle / acide acrylique, lesdits copolymères contenant entre 70 et 90 % en poids d'acrylate d'isobornyle ;
- Les copolymères de méthacrylate de norbornyle/ acide méthacrylique ; les copolymères de méthacrylate de norbornyle / acide acrylique, lesdits copolymères contenant entre 70 et 90 % en poids de méthacrylate de norbornyle ;
- Les copolymères d'acrylate de norbornyle / acide méthacrylique ; les copolymères d'acrylate de norbornyle / acide acrylique, lesdits copolymères contenant entre 70 et 90 % en poids d'acrylate de norbornyle ; et
- Les copolymères de styrène / acide méthacrylique ; les copolymères de styrène / acide acrylique, lesdits copolymères

contenant entre 70 et 90 % en poids de styrène.

*Monomère de Tg < 40 °C*

Selon un mode de réalisation particulier, les copolymères comportant en outre au moins une « unité monomère » issue d'un monomère dont l'homopolymère correspondant présente une température de transition vitreuse inférieure à 40 °C (et notamment supérieure à -100 °C) font également partie de l'invention pour autant que la température globale de transition vitreuse dudit copolymère reste bien supérieure ou égale à 45 °C.

Selon un autre mode de réalisation particulier, les monomères additionnels, dont les homopolymères présentent une température de transition vitreuse inférieure à 40 °C, dits « monomères de Tg < 40 °C » diffèrent des monomères hydrophobes et des monomères hydrophiles ioniques décrits précédemment.

La teneur en monomères dont l'homopolymère présente une température de transition vitreuse inférieure à 40 °C est ajustée de sorte que l'effet tenseur du copolymère ne se trouve pas affecté.

La teneur dans le copolymère est avantageusement inférieure ou égale à 25 % en poids, par rapport au poids total du copolymère (notamment de 0 à 25 % en poids, et de préférence de 5 à 25 % en poids), de manière encore plus préférée inférieure ou égale à 10 % en poids (notamment de 0 à 10 % en poids, et de préférence de 5 à 10 % en poids).

Les monomères dont les homopolymères ont des températures de transition vitreuse inférieure à 40 °C sont choisis parmi les monomères suivants :

- les hydrocarbures éthyléniques en $C_2$ à $C_{10}$, tels que l'éthylène, l'isoprène et le butadiène.
- les acrylates de formule

$$CH_2 = CHCOOR_{12}$$

où $R_{12}$ représente :

- un groupe alkyle en $C_1$ à $C_{12}$ linéaire ou ramifié (à l'exception du groupe tertiobutyle), dans lequel se trouve(nt) éventuellement intercalé(s) un ou plusieurs hétéroatomes identiques ou non, ledit groupe alkyle pouvant en outre être éventuellement substitué par un ou plusieurs substituants identiques ou non choisis parmi les groupes hydroxyle et les atomes d'halogène (notamment fluor), de préférence un groupe alkyle en $C_1$ à $C_{12}$ linéaire ou ramifié (à l'exception du groupe tertiobutyle); des exemples de groupes $R_{12}$ sont les groupes méthyle, éthyle, propyle, butyle, isobutyle, hexyle, éthylhexyle, octyle, lauryle, isooctyle, isodécyle, hydroxyéthyle, hydroxypropyle, méthoxyéthyle, éthoxyéthyle et méthoxypropyle ;
- un groupe alkyle en $C_1$ à $C_{12}$ - POE (polyoxyéthylène), avec répétition du motif oxyéthylène de 5 à 30 fois, par exemple méthoxy POE, $R_{12}$ pouvant alors être représenté par $R_{13}$-$(OC_2H_4)_n$-, où $R_{13}$ est un groupe alkyle en $C_1$ à $C_{12}$, et n est un entier variant de 5 à 30 ; ou
- un groupement polyoxyéthylène de structure $-(CH_2CH_2O)_n-H$ où n est un entier variant de 5 à 30.
- les méthacrylates de formule

$$CH_2 = C(CH_3)COOR_{14}$$

où $R_{14}$ représente :

- un groupe alkyle en $C_4$ à $C_{12}$ linéaire ou un groupe alkyle en $C_5$ à $C_{12}$ ramifié (à l'exception des groupes cycloalkyle, aryle, aralkyle, hétérocycloalkyle et hétéroaralkyle), dans lequel se trouve(nt) éventuellement intercalé(s) un ou plusieurs hétéroatomes identiques ou non, ledit groupe alkyle pouvant en outre être éventuellement substitué par un ou plusieurs substituants identiques ou non choisis parmi les groupes hydroxyle et les atomes d'halogènes ; et de préférence un groupe alkyle en $C_4$ à $C_{12}$ linéaire ou un groupe en $C_5$ à $C_{12}$ ramifié non substitué ; des exemples de groupes $R_{14}$ sont les groupes héxyle, éthylhéxyle, octyle, lauryle, isooctyle, isodécyle, dodécyle, méthoxyéthyle, méthoxypropyle et éthoxyéthyle,
- un groupe alkyle en $C_1$ à $C_{12}$ - POE (polyoxyéthylène), avec répétition du motif oxyéthylène de 5 à 30 fois, par exemple méthoxy POE, $R_{14}$ pouvant alors être représenté par $R_{13}$-$(OC_2H_4)_n$-, où $R_{13}$ est un groupe alkyle en $C_1$ à $C_{12}$, et n est un entier variant de 5 à 30 ; ou
- un groupement polyoxyéthylène de structure $-(CH_2CH_2O)_n-H$ où n est un entier variant de 5 à 30.

Les monomères, particulièrement préférés, sont : l'acrylate de méthyle, l'acrylate d'éthyle, l'acrylate de propyle, l'acrylate de n-butyle, l'acrylate de 2-éthylhexyle, l'acrylate d'isobutyle, l'acrylate de méthoxyéthyle, le (méth)acrylate d'éthoxyéthyle, le (méth)acrylate de n-hexyle.

- les esters vinyliques de formule

$R_{15}COOCH = CH_2$

où $R_{15}$ représente un groupe alkyle en $C_2$ à $C_{12}$ linéaire ou ramifié.

Des exemples de tels esters vinyliques sont : le propionate de vinyle, le butyrate de vinyle, l'éthylhexanoate de vinyle, le néononanoate de vinyle, et le néododécanoate de vinyle.

- Les éthers d'alcool vinylique et d'alcool en $C_1$ à $C_{12}$, tels que le méthyle vinyle éther et l'éthyle vinyle éther.

Le monomère additionnel est de préférence choisi parmi les acrylates et méthacrylates décrits précédemment, et de préférence parmi les acrylates d'alkyle en $C_1$-$C_{12}$, et les méthacrylates d'alkyle en $C_4$-$C_{12}$.
Plus particulièrement, les monomères additionnels préférés sont :

- les acrylates de formule :

  $CH_2 = CHCOOR_{12}$

  où $R_{12}$ représente un groupe alkyle en $C_1$-$C_{12}$ linéaire ou ramifié (à l'exception du groupe tertiobutyle), dans lequel se trouve(n) éventuellement intercalé(s) un ou plusieurs hétéroatomes identiques ou non, ledit groupe alkyle pouvant en outre être éventuellement substitué par un ou plusieurs substituants identiques ou non choisis parmi les groupes hydroxyle et les atomes d'halogène, et
- les méthacrylates de formule :

  $CH_2 = C(CH_3)COOR_{14}$

où $R_{14}$ représente un groupe alkyle en $C_4$ à $C_{12}$ linéaire ou un groupe alkyle en $C_5$ à $C_{12}$ ramifié.
Selon un mode de réalisation de l'invention, le monomère additionnel est l'un des monomères détaillés ci-dessus, à l'exclusion de l'acrylate d'éthylhexyle.

COMPOSITION

La teneur exprimée en matière sèche en copolymère(s) présent(s) dans une composition selon la présente invention peut aller de 0,1 à 15 %, de préférence de 1 à 10 % et de façon encore plus préférée de 1 à 5 % en poids par rapport au poids total de la composition.
La composition cosmétique selon la présente invention se présente sous la forme d'une émulsion eau-dans-huile ou d'une émulsion multiple.
Par ailleurs, dans le cadre de la présente invention, le terme « émulsion multiple » couvre les émulsions eau-dans-huile-dans-eau, huile-dans-eau-dans-huile, et eau-dans-huile-de-silicone-dans-eau, l'émulsion multiple eau-dans-huile-dans-eau étant préférée.

**Emulsions inverses eau-dans-huile (E/H)**

Selon un premier de ses aspects, la composition selon l'invention peut se trouver sous la forme d'une émulsion eau-dans-huile E/H.
La composition selon l'invention peut être préparée selon toute méthode connue de l'homme du métier pour préparer des émulsions eau-dans-huile E/H. Il peut avantageusement s'agir d'une émulsion eau-dans-huile E/H, comprenant une phase aqueuse interne gélifiée ou non et une phase grasse comprenant au moins une huile et au moins un tensioactif.

*Phase aqueuse*

La phase aqueuse forme la phase dispersée de l'émulsion E/H selon l'invention.
La phase aqueuse comprend de l'eau et peut être constituée essentiellement d'eau.
Elle peut également comprendre un mélange d'eau et de solvant organique miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol,

le 1,3-butylène glycol, le dipropylène glycol, les cétones en $C_3$-$C_4$, et les aldéhydes en $C_2$-$C_4$.

La phase aqueuse (eau et éventuellement le solvant organique miscible à l'eau) peut être présente en une teneur allant de 1 % à 95 % en poids, notamment allant de 5 % à 80 % en poids, et en particulier allant de 15 % à 60 %, en poids par rapport au poids total de la composition sous forme d'émulsion eau-dans-huile.

La phase aqueuse interne peut être gélifiée ou non gélifiée. Selon un mode de réalisation préféré, la phase aqueuse interne est non gélifiée.

*Phase grasse*

La phase grasse forme la phase continue de l'émulsion E/H selon l'invention.

Elle comprend au moins un corps gras liquide à température ambiante (25 °C) tel qu'une huile, le cas échéant associé à un corps gras solide à température ambiante tel que les cires, les corps gras pâteux, les gommes et leurs mélanges. La phase grasse peut en outre contenir des solvants organiques lipophiles.

La teneur en phase grasse peut aller de 5 à 80 % en poids, et de préférence de 5 à 50 % en poids par rapport au poids total de la composition sous forme d'émulsion eau-dans-huile.

*Huile*

La phase grasse de la composition selon l'invention peut notamment comprendre, à titre de corps gras liquide, au moins une huile volatile ou non volatile ou un de leurs mélanges.

Selon un mode de réalisation préféré, la composition conforme à l'invention comprend au moins une huile siliconée, volatile ou non.

Par « huile volatile », on entend au sens de l'invention toute huile susceptible de s'évaporer au contact de la peau en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,01 à 300 mm de Hg (1,33 Pa à 40000 Pa) et de préférence supérieure à 0,3 mm de Hg (30 Pa).

Par « huile non volatile », on entend une huile restant sur la peau à température ambiante et pression atmosphérique au moins plusieurs heures et ayant notamment une pression de vapeur inférieure à 0,01 mm de Hg (1,33 Pa).

Ces huiles volatiles ou non volatiles peuvent être des huiles hydrocarbonées, des huiles siliconées, ou leurs mélanges.

Par « huile hydrocarbonée », on entend une huile contenant principalement des atomes d'hydrogène et de carbone et éventuellement des atomes d'oxygène, d'azote, de soufre, de phosphore, et ne contenant pas d'atome de silicium ou de fluor. Elle peut contenir des groupes alcool, ester, acide carboxylique, amine et/ou amide.

Les huiles hydrocarbonées volatiles peuvent être choisies parmi les huiles hydrocarbonées ayant de 8 à 16 atomes de carbone, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars® ou de Permetyls®, les esters ramifiés en $C_8$-$C_{16}$ tels que le néopentanoate d'iso-hexyle, et leurs mélanges.

D'autres huiles hydrocarbonées volatiles comme les distillats de pétrole, notamment ceux vendus sous la dénomination Shell Solt® par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité ≤ 8 centistokes ($8 \times 10^{-6}$ $m^2/s$), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

Les huiles non volatiles peuvent notamment être choisies parmi les huiles hydrocarbonées fluorées et/ou siliconées non volatiles.

Comme huile hydrocarbonée non volatile, on peut notamment citer :

- les huiles hydrocarbonées d'origine animale, comme l'huile de vison, de tortue, le perhydrosqualène,
- les huiles hydrocarbonées d'origine végétale telles que les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron,

de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810, 812 et 818® par la société Dynamit Nobel,

- les éthers de synthèse ayant de 10 à 40 atomes de carbone ;
- les hydrocarbures linéaires ou ramifiés, d'origine minérale ou synthétique tels que l'huile de paraffine ou ses dérivés, la vaseline, les polydécènes, le polyisobutène hydrogéné tel que le Parléam® commercialisé par la société NIPPON OIL FATS, le squalane, et leurs mélanges,
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit ≥ 10, comme par exemple l'huile de Purcellin (octanoate de cétostéaryle), le myristate d'isopropyle, le palmitate d'isopropyle, les benzoates d'alcools en $C_{12}$ à $C_{15}$, le laurate d'hexyle, l'adipate de diisopropyle, l'isononanoate d'isononyle, le palmitate de 2-éthyl-hexyle, l'isostéarate d'isostéaryle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol,
- les acides gras supérieurs en $C_8$-$C_{26}$ tels que l'acide oléique, l'acide linoléique, l'acide linolénique ou l'acide isostéarique et leurs mélanges,
- les esters d'acide gras, en particulier de 4 à 22 atomes de carbone, et notamment d'acide octanoïque, d'acide heptanoïque, d'acide lanolique, d'acide oléique, d'acide laurique, d'acide stéarique comme le dioctanoate de propylène glycol, le monoisostéarate de propylène glycol, le poly-glycéryl 2-diisostéarate, le diheptanoate de néopentylglycol,
- les esters hydroxylés comme le lactate d'isostéaryle, l'octylhydroxystéarate, l'hydroxystéarate d'octyldodécyle, le diisostéarylmalate, le citrate de triisocétyle, le triisostéarate de glycérine ou de diglycérine; le diisononanoate de diéthylèneglycol ; et
- les esters du pentaérythritol ; les esters d'acides aromatiques et d'alcools comprenant 4 à 22 atomes de carbone, notamment le trimellitate de tridécyle,
- les alcools gras liquides à température ambiante à chaîne carbonée ramifiée et/ou insaturée ayant de 8 à 26 atomes de carbone comme l'alcool oléique, l'alcool linoléique ou linolénique, l'alcool isostéarique ou l'octyl dodécanol ;
- les huiles fluorées,
- et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates et leurs mélanges.

Les huiles non volatiles peuvent être présentes dans une composition selon l'invention en une teneur allant de 0,01 à 80 % en poids, notamment de 0,1 à 75 % en poids, et en particulier de 1 % à 70 % en poids, par rapport au poids total de la composition sous forme d'émulsion eau-dans-huile.

Les huiles volatiles peuvent être présentes dans une composition selon l'invention en une teneur allant de 0,01 à 80 % en poids, notamment de 0,1 à 75 % en poids, et en particulier de 1 % à 70 % en poids, par rapport au poids total de la composition sous forme d'émulsion eau-dans-huile.

**Corps gras solides**

La phase grasse de la composition selon l'invention peut comprendre au moins un corps gras solide à température ambiante et à pression atmosphérique, il peut être choisi parmi les cires, les corps gras pâteux, les gommes et leurs mélanges. Ce corps gras solide peut être présent à raison de 0,01 à 50 %, notamment de 0,1 à 25 % et en particulier de 0,2 à 20 % en poids, par rapport au poids total de la composition sous forme d'émulsion eau-dans-huile.

*Corps gras pâteux*

Ainsi, la composition selon l'invention peut comprendre au moins un composé gras pâteux à température ambiante.

Par « corps gras pâteux » au sens de l'invention, on entend des corps gras ayant un point de fusion allant de 20 à 55 °C, de préférence 25 à 45 °C, et/ou une viscosité à 40 °C allant de 0,1 à 40 Pa.s (1 à 400 poises), de préférence 0,5 à 25 Pa.s, mesurée au Contraves TV ou Rhéomat 80, équipé d'un mobile tournant à 60 Hz. L'homme du métier peut choisir le mobile permettant de mesurer la viscosité, parmi les mobiles MS-r3 et MS-r4, sur la base de ses connaissances générales, de manière à pouvoir réaliser la mesure du composé pâteux testé.

Plus particulièrement, ces corps gras sont des composés hydrocarbonés, éventuellement de type polymérique ; ils peuvent également être choisis parmi les composés siliconés; ils peuvent aussi se présenter sous forme d'un mélange de composés hydrocarbonés et/ou siliconés. Dans le cas d'un mélange de différents corps gras pâteux, on utilise de préférence les composés pâteux hydrocarbonés (contenant principalement des atomes de carbone et d'hydrogène et éventuellement des groupements ester), en proportion majoritaire.

Parmi les composés pâteux susceptibles d'être utilisés dans la composition selon l'invention, on peut citer les lanolines et les dérivés de lanoline comme les lanolines acétylées, les lanolines oxypropylènées ou le lanolate d'isopropyle, ayant une viscosité de 18 à 21 Pa.s, de préférence 19 à 20,5 Pa.s, et/ou un point de fusion de 30 à 55 °C et leurs mélanges. On peut également utiliser des esters d'acides ou d'alcools gras, notamment ceux ayant 20 à 65 atomes de carbone (point de fusion de l'ordre de 20 à 35 °C et/ou viscosité à 40 °C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux comme l'acide poly (12-hydroxystéarique) et leurs mélanges. Comme triglycéride d'origine végétale, on peut utiliser les dérivés d'huile de ricin hydrogénée, tels que le « THIXINR » de Rheox.

On peut aussi citer les corps gras pâteux siliconés tels que les polydiméthylsiloxanes (PDMS) de hauts poids moléculaires et en particulier ceux ayant des chaînes pendantes du type alkyle ou alcoxy ayant de 8 à 24 atomes de carbone, et un point de fusion de 20-55 °C, comme les stéaryl diméthicones notamment ceux vendus par la société Dow Corning sous les noms commerciaux de DC2503® et DC25514®, et leurs mélanges.

Le corps gras pâteux peut être présent dans une composition selon l'invention en une teneur allant de 0,01 à 50 % en poids, de préférence allant de 0,1 à 45 % en poids, et mieux allant de 0,2 % à 30 % en poids, par rapport au poids total de la composition sous forme d'émulsion eau-dans-huile.

*Cires*

La phase grasse peut comprendre en outre une cire ou un mélange de cires. La cire peut être solide à température ambiante (25 °C), à changement d'état solide/liquide réversible, ayant une température de fusion supérieure à 30 °C pouvant aller jusqu'à 120 °C. Elle peut être hydrocarbonée, fluorée et/ou siliconée et être d'origine animale, végétale, minérale ou synthétique.

On peut notamment utiliser les cires hydrocarbonées comme la cire d'abeilles, la cire de lanoline, et les cires d'insectes de Chine ; la cire de riz, la cire de Carnauba, la cire de Candellila, la cire d'Ouricurry, la cire d'Alfa, la cire de fibres de liège, la cire de canne à sucre, la cire du Japon et la cire de sumac ; la cire de montan, les cires microcristallines, les paraffines et l'ozokérite ; les cires de polyéthylène, les cires obtenues par la synthèse de Fisher-Tropsch et les copolymères cireux ainsi que leurs esters et leurs mélanges

On peut aussi citer les cires obtenues par hydrogénation catalytique d'huiles animales ou végétales ayant des chaînes grasses, linéaires ou ramifiées, en $C_8$-$C_{32}$.

Parmi celles-ci, on peut notamment citer l'huile de jojoba hydrogénée, l'huile de jojoba isomérisée telle que l'huile de jojoba partiellement hydrogénée isomérisée trans-fabriquée ou commercialisée par la société Desert Whale sous la référence commerciale ISO-JOJOBA-50®, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination HEST 2T-4S® par la société HETERENE, le tétrabéhénate de di-(triméthylol-1,1,1 propane) vendue sous la dénomination HEST 2T-4B® par la société HETERENE.

On peut encore citer les cires de silicone, les cires fluorées.

On peut également utiliser la cire obtenue par hydrogénation d'huile d'olive estérifiée avec l'alcool stéarylique vendue sous la dénomination PHYTOWAX Olive 18 L57® ou bien encore les cires obtenues par hydrogénation d'huile de ricin estérifiée avec l'alcool cétylique vendus sous la dénomination PHYTOWAX ricin 16L64® et 22L73® par la société SOPHIM. De telles cires sont décrites dans la demande FR-A-2792190.

La cire peut être présente dans une composition selon l'invention en une teneur allant de 0,01 % à 50 % en poids, en particulier de 0,1 % à 30 % en poids, et notamment de 0,2 % à 20 % en poids, par rapport au poids total de la composition sous forme d'émulsion eau-dans-huile.

**Agents tensioactifs**

L'émulsion contient avantageusement au moins un agent tensioactif qui peut être présent notamment en une

proportion allant de 0,1 à 30 % en poids, et mieux de 5 % à 15 % en poids, par rapport au poids total de la composition sous forme d'émulsion eau-dans-huile.

L'agent tensioactif de ces émulsions est généralement choisi parmi ceux du type hydrocarboné, siliconé et/ou fluoré.

L'agent tensioactif peut être un composant unique ou un mélange d'agents tensioactifs ; notamment un mélange d'agents tensioactifs choisis parmi ceux cités ci après.

Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions des tensioactifs, en particulier p.347-377 de cette référence, pour les tensioactifs anioniques et non ioniques.

Les tensioactifs permettant l'obtention d'émulsions eau-dans-huile sont les tensioactifs donc la HLB (balance hydrophile / lipophile) est comprise entre 3 et 6. La définition de la HLB figure dans le livre Galenica 5, Les Systèmes Dispersés-I Agents de Surface et Emulsions, F. Puisieux, M. Seiller, Pages 153-155, Editions Lavoisier.

Selon une première variante, les agents tensioactifs sont des tensioactifs siliconés.

Les tensioactifs siliconés utilisables dans les compositions cosmétiques sous forme d'émulsion eau-dans-huile selon l'invention peuvent en particulier être choisis parmi les diméthicone copolyols, les alkyl-ou alcoxy-diméthicone copolyols, (en particulier les cétyl diméthicone copolyols) et leurs mélanges.

Ils peuvent notamment être choisis parmi les alkyldiméthicones copolyols et les diméthicone copolyols.

Les tensioactifs utilisables selon cette première variante de l'invention, adaptés à l'obtention d'une émulsion E/H, peuvent être choisis parmi les diméthicone copolyols tels que le mélange de cyclométhicone et de diméthicone copolyol, par exemple vendu sous la dénomination « DC 5225 C » par la société Dow Corning, et les diméthicone copolyols tels que le laurylmethicone copolyol par exemple vendu sous la dénomination Dow Corning 5200 Formulation Aid® par la société Dow Corning et le Cetyl diméthicone copolyol par exemple vendu sous la dénomination ABIL EM 90R® par la société Goldschmidt, ou le mélange polyglycéryl-4 isostéarate/cétyl diméthicone copolyol/hexyllaurate par exemple vendu sous la dénomination ABIL WE 09® par la société Goldschmidt et leurs mélanges.

Parmi ces tensioactifs siliconés, on peut encore mentionner ceux vendus sous les dénominations ABIL WS08® ou ABIL EM97® par la société GOLDSCHMIDT, DC-5200®, Q2-3225® par la société DOW CORNING et 218-1138®, SF 1228® ou SF1328® par la société GENERAL ELECTRIC.

Selon une deuxième variante, les tensioactifs adaptés à l'obtention d'une émulsion eau-dans-huile E/H peuvent être sélectionnés parmi le groupe comprenant les tensioactifs polyisobutylene à terminaison succinique estérifiée, tels que ceux commercialisés sous les noms de Lubrizol 5603® et Chemcinnate 2000® par les sociétés Lubrizol et Chemron (voir FR2811565 et W004100904).

Selon une troisième variante, les tensioactifs d'émulsions eau-dans-huile E/H sont du type élastomère de silicone émulsionnant.

Par « élastomère de silicone émulsionnant », on entend au sens de l'invention un élastomère de silicone comprenant au moins une chaîne hydrophile, et qui peut être choisi parmi les élastomères de silicone polyoxyalkylénés.

L'élastomère de silicone polyoxyalkyléné est un organopolysiloxane réticulé pouvant être obtenu par réaction d'addition-réticulation d'un diorganopolysiloxane contenant au moins un hydrogène lié au silicium et d'un polyoxyalkylène ayant au moins deux groupements à insaturation éthylénique.

L'élastomère de silicone polyoxyalkyléné est généralement véhiculé sous forme de gel dans au moins une huile hydrocarbonée et/ou une huile siliconée. Des élastomères polyoxyalkylénés sont notamment décrits dans les brevets US 5 236 986, US 5 412 004, US 5 837 793, US 5 811 487 dont le contenu est incorporé par référence.

Comme élastomères de silicone polyoxyalkyléné convenant à la mise en oeuvre de la présente invention, on peut notamment citer ceux commercialisés sous les dénominations « KSG-21 », « KSG-20 », « KSG-30 », « KSG-31 », « KSG-32 », « KSG-33 », « KSG-210 », « KSG-310 », « KSG-320 », « KSG-330 », « KSG-340 », « X-226146 » par la société SHIN ETSU, ou encore « DC9010 » et « DC9011 » par la société DOW CORNING.

On peut ajouter aux agents tensioactifs cités ci-dessus un ou plusieurs co-émulsionnants, qui peuvent, de manière avantageuse, être choisi(s) dans le groupe comprenant les esters alkylés de polyol. Comme esters alkylés de polyol, on peut citer notamment les esters de glycérol et/ou de sorbitane et par exemple l'isostéarate de polyglycérol, tel que le produit commercialisé sous la dénomination Isolan GI 34® par la société Goldschmidt, l'isostéarate de sorbitane, tel que le produit commercialisé sous la dénomination Arlacel 987® par la société ICI, l'isostéarate de sorbitane et de glycérol, tel que le produit commercialisé sous la dénomination Arlacel 986® par la société ICI, et leurs mélanges.

Le tensioactif et le coémulsionnant sont de préférence présents, dans la composition, en une teneur totale allant de 0,3 à 30 % en poids, et de préférence de 0,5 à 20 % en poids par rapport au poids total de la composition sous forme d'émulsion eau-dans-huile.

Selon un mode de réalisation particulier, les émulsions eau-dans-huile selon l'invention sont obtenues suivant un procédé de préparation particulier qui consiste à introduire le copolymère statistique à chaîne principale linéaire de nature éthylénique à effet tenseur tel que défini précédemment après la réalisation de l'émulsion.

Selon un mode de réalisation préféré, le copolymère statistique conforme à l'invention est introduit dans la composition avec la phase aqueuse, au cours de l'étape d'émulsification.

**Emulsions multiples**

Selon un autre de ses aspects, la composition selon l'invention peut se trouver sous la forme d'une émulsion multiple, par exemple sous la forme d'une émulsion eau-dans-huile-dans-eau, huile-dans-eau-dans-huile, ou eau-dans-huile-de-silicone-dans-eau.

Selon un mode de réalisation préféré, l'émulsion multiple est du type eau-dans-huile-dans-eau.

Comme émulsions eau-dans-huile-de-silicone-dans-eau convenant à la mise en oeuvre de la présente invention, on peut par exemple citer celles décrites dans le brevet EP 0 648 102.

Selon un mode de réalisation particulier, la phase aqueuse externe de l'émulsion eau-dans-huile-de-silicone-dans-eau est gélifiée.

Dans le cas d'une émulsion multiple eau-dans-l'huile-dans-l'eau (E/H/E), la composition selon l'invention peut être préparée selon toute méthode connue de l'homme du métier.

Il peut s'agir notamment d'une émulsification d'une émulsion eau-dans-huile (E/H), dite émulsion primaire, dans une phase aqueuse gélifiée ou non, dite phase aqueuse externe.

Selon un mode de réalisation de l'invention, l'émulsion multiple est obtenue par émulsification d'une émulsion eau-dans-huile dans une phase aqueuse gélifiée.

L'émulsion primaire peut en particulier être conforme à une émulsion eau-dans-huile E/H telle que décrite précédemment.

*Phase aqueuse externe*

Pour assurer l'émulsification de l'émulsion primaire dans la phase aqueuse externe continue de l'émulsion, celle-ci peut contenir, selon une première variante, au moins un copolymère tensioactif constitué d'une fraction majoritaire d'un monomère acide carboxylique monooléfiniquement insaturé en $C_3$-$C_6$ ou de son anhydride et d'une fraction minoritaire de monomère ester gras d'acide acrylique. De tels copolymères sont décrits dans la demande EP 0 268 164 et sont obtenus selon les méthodes de préparation décrites dans ce même document. On peut par exemple utiliser un polymère à chaîne grasse du type copolymère acide ou anhydre d'acide carboxylique monoéthylénique en $C_3$-$C_6$/ester acrylique à chaîne grasse.

Ce polymère à chaîne grasse peut être notamment choisi parmi les copolymères commercialisés sous les dénominations PEMULEN® et CARBOPOL 1342® ou CARBOPOL 1382® par la société GOODRICH.

Dans un mode de réalisation particulier, il peut s'agir plus particulièrement d'un copolymère acrylate/$C_{10}$-$C_{30}$-alkylacrylate, et notamment celui vendu sous la dénomination PEMULEN TR1® ou PEMULEN TR2® par la société GOODRICH.

Ce type d'émulsion triple est notamment décrit dans les demandes EP 0 908 170 et EP 0 648 102.

Dans une émulsion multiple E/H/E, l'eau interne en dispersion dans la phase grasse se trouve séparée de l'eau externe par une membrane d'huile. Pour assurer la stabilité du système, il peut être nécessaire de gélifier l'une ou l'autre partie afin d'éviter la fuite de l'eau interne vers l'eau externe.

Comme gélifiants hydrophiles, on peut citer en particulier les polymères carboxyvinyliques (carbomer), les copolymères acryliques tels que les copolymères d'acrylates/alkylacrylates, les polyméthacrylates de glycéryle, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme exemple de polymères carboxyvinyliques, on peut citer ceux vendus sous les dénominations CARBOPOL 980®, CARBOPOL 942® ou CARBOPOL 950® par la société GOODRICH, ou celui vendu sous la dénomination SYNTHALEN K® par la société SIGMA.

Selon une autre forme de réalisation de l'invention, on utilise une association de deux gélifiants dans la phase aqueuse externe, constitués d'une part d'un sel de métal alcalin d'un copolymère acrylique vendu sous la dénomination HOSTACERINE PN 73® par la société HOECHST, et d'autre part, d'un polyglycéryl méthacrylate fabriqué sous la dénomination LUBRAGEL MS® par la société UNITED GUARDIAN INC.

Selon une deuxième variante, la phase aqueuse externe continue de l'émulsion peut contenir à titre de gélifiant un polymère ou copolymère d'acide acrylique ou méthacrylique associé à un polyglycéryl méthacrylate, et la phase grasse peut renfermer une huile fluorée. Ce type d'émulsion triple est notamment décrit dans la demande EP 0 507 693. Avantageusement la seconde phase aqueuse gélifiée des compositions selon cette deuxième variante comporte un agent tensioactif choisi de préférence parmi les esters de sucrose.

Selon une troisième variante, l'une des phases aqueuses de l'émulsion eau-dans-huile-dans-eau E/H/E peut avoir une valeur d'activité en eau inférieure ou égale à 0,85.

L'activité en eau $a_w$ d'un milieu contenant de l'eau est le rapport de la pression de vapeur d'eau du milieu « $P_{H2O}$ milieu » et de la pression de vapeur de l'eau pure « $P_{H2O}$ pur » à la même température. Elle peut être exprimée aussi comme le rapport du nombre de molécules d'eau « $N_{H2O}$ » sur le nombre de molécules totales « $H_{H2O}$ + $N_{corps\ dissous}$ » qui tient compte de celles des corps dissous « $N_{corps\ dissous}$ ».

Elle est donnée par les formules suivantes :

$$a_w = \frac{P_{H2O}\,milieu}{P_{H2O}\,pur} = \frac{N_{H2O}}{N_{H2O} + N_{corps\,dissous}}$$

On peut utiliser différentes méthodes pour mesurer l'activité en eau. La plus courante est la méthode manométrique par laquelle on mesure directement la pression de vapeur.

De manière classique, une composition cosmétique ou dermatologique a une activité en eau située autour de 0,95 à 0,99. Une activité en eau inférieure à 0,85 représente une diminution notable de l'activité en eau.

Une valeur d'activité en eau inférieure ou égale à 0,85 peut notamment être obtenue par incorporation d'une quantité efficace de polyol comme décrit dans la demande EP 0 779 071. Pour avoir une telle activité en eau inférieure à 0,85, la quantité de polyol dans la phase considérée, de préférence la phase aqueuse externe continue, doit aller de 35 à 90 % en poids, et mieux de 60 à 85 % en poids par rapport au poids total de la phase aqueuse à faible activité en eau, c'est-à-dire être supérieure à 30 % en poids par rapport au poids total de l'émulsion, et de préférence aller de 35 à 70 % en poids par rapport au poids total de l'émulsion. Comme polyol utilisable pour la réalisation de telles émulsions on peut notamment citer la glycérine et les glycols, en particulier le propylène glycol et les polyéthylène glycols. Selon un mode de réalisation préféré, le ou les polyols se trouvent totalement ou partiellement sous forme complexée avec un polymère acrylique ou méthacrylique.

La teneur en copolymère tensioactif tel que décrit ci-dessus dans l'émulsion selon l'invention peut aller de 0,05 à 3 % en poids, de préférence de 0,1 à 2 % en poids par rapport au poids total de l'émulsion multiple.

De préférence, l'émulsion primaire est réalisée en suivant le procédé consistant à introduire dans l'émulsion le copolymère statistique à effet tenseur selon l'invention sous forme de dispersion aqueuse, avec la phase aqueuse interne.

La composition de l'invention constitue plus particulièrement une composition anti-âge, en particulier anti-rides. Elle peut toutefois, en variante, constituer une composition de soin du corps, et en particulier une composition amincissante.

**Adjuvants**

De façon connue, la composition de l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les polymères filmogènes, les filtres, les pigments, les absorbeurs d'odeur et les matières colorantes. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules. Ces adjuvants ainsi que leurs concentrations doivent être tels qu'ils ne modifient pas la propriété recherchée de tenseur du copolymère statistique à chaîne principale linéaire de nature éthylénique.

En outre, on peut aussi associer aux agents tenseurs utilisés selon l'invention d'autres composés connus de l'homme du métier comme agents tenseurs et ayant des propriétés différentes de celles des agents utilisés selon l'invention, notamment les latex synthétiques, les protéines végétales, les polysaccharides d'origine végétale sous forme ou non de microgels, les amidons, les silicates mixtes et les particules colloïdales de charges inorganiques.

En variante ou en plus, lorsque la composition selon l'invention est une composition amincissante, on préfère qu'elle comprenne, dans un milieu physiologiquement acceptable, au moins un copolymère statistique à chaîne principale linéaire de nature éthylénique tel que défini précédemment et un ou plusieurs composés drainants, lipolytiques, désinfiltrants, amincissants, raffermissants, anti-glycants et/ou vaso-protecteurs.

La quantité d'actif(s) amincissant(s) peut varier dans une large mesure et dépend de la nature de ou des actifs utilisés. De manière générale, le ou les actifs amincissants sont présents en une concentration allant de 0,05 à 20 % et de préférence de 0,1 à 10 % en poids par rapport au poids total de la composition.

Lorsque la composition selon l'invention constitue une composition anti-âge, il sera avantageux d'introduire dans cette composition au moins un composé choisi parmi : les agents desquamants ; les agents hydratants ; les agents dépigmentants ou propigmentants ; les agents anti-glycation ; les inhibiteurs de NO-synthase ; les agents stimulant la synthèse de macromolécules dermiques ou épidermiques et/ou empêchant leur dégradation ; les agents stimulant la

prolifération des fibroblastes et/ou des kératinocytes ou stimulant la différenciation des kératinocytes ; les autres agents myorelaxants et/ou dermo-décontractants ; les agents tenseurs ; les agents anti-pollution et/ou anti-radicalaire ; les agents agissant sur la microcirculation ; les agents agissant sur le métabolisme énergétique des cellules ; et leurs mélanges.

Des exemples de tels composés additionnels sont : le rétinol et ses dérivés tels que le palmitate de rétinyle ; l'acide ascorbique et ses dérivés tels que l'ascorbyl phosphate de magnésium et le glucoside d'ascorbyle ; le tocophérol et ses dérivés tels que l'acétate de tocophéryle ; l'acide nicotinique et ses précurseurs tels que la nicotinamide ; l'ubiquinone ; le glutathion et ses précurseurs tels que l'acide L-2-oxothiazolidine-4-carboxylique ; les extraits de plantes et notamment les protéines végétales et leurs hydrolysats, ainsi que les phytohormones ; les extraits marins tels que les extraits d'algues ; les extraits bactériens ; les sapogénines telles que la diosgénine et les extraits de Wild Yam en contenant ; les céramides ; les hydroxyacides ; les hydroxyacides, tels que l'acide salicylique et l'acide n-octanoyl-5-salicylique ; le resvératrol ; les oligopeptides et pseudodipeptides et leurs dérivés acylés ; les sels de manganèse et de magnésium, en particulier les gluconates ; et leurs mélanges.

La quantité de ces actifs peut varier dans une large mesure. De manière générale, ces actifs sont présents en une concentration allant de 0,01 à 15 % et de préférence de 0,05 à 10 % en poids par rapport au poids total de la composition.

En cas d'incompatibilité, les actifs indiqués ci-dessus peuvent être incorporés dans des sphérules, notamment des vésicules ioniques ou non-ioniques et/ou des nanoparticules (nanocapsules et/ou nanosphères), de manière à les isoler les uns des autres dans la composition.

La composition selon l'invention peut également être associée à une autre composition cosmétique.

Ainsi, la présente invention a en outre pour objet un produit cosmétique comprenant au moins :

- une première composition, sous forme d'émulsion eau-dans-huile ou d'émulsion multiple selon l'invention, contenant au moins un copolymère statistique à chaîne principale linéaire de nature éthylénique tel que défini précédemment, et
- une deuxième composition comprenant au moins un milieu physiologiquement acceptable.

La première composition du produit selon l'invention peut constituer généralement une couche de base appliquée sur la matière kératinique et la seconde composition une couche de dessus ou « top coat ». Il est toutefois possible d'appliquer sur la seconde couche une surcouche ayant la constitution ou non de la seconde couche.

Le produit selon l'invention peut comprendre deux (ou plusieurs) compositions physiologiquement acceptables conditionnées séparément ou ensemble dans un même article de conditionnement ou dans deux (ou plusieurs) articles de conditionnement séparés ou distincts.

Selon un autre de ses aspects, la présente invention concerne un kit de compositions comprenant un produit selon l'invention.

De préférence, ces compositions sont conditionnées séparément et avantageusement dans des compartiments ou récipients séparés ou distincts.

Selon un mode de réalisation particulier, ces compositions peut être conditionnées sous la forme d'un ensemble de distribution double pompe, comme par exemple ceux décrits dans les brevets US 5,692,644, US 5,884,759, US 5,875,888, US 5,875,889, US 5,992,693, US 5,944,175 et US 6,402,364.

Les exemples ci-après de compositions selon l'invention sont donnés à titre d'illustration et sans caractère limitatif. Les quantités y sont données en pourcentages en poids. Les taux de nautralisation des copolymères sont de 100 %.

Dans les exemples qui suivent « Mp » est l'abréviation pour masse moléculaire au pic ; « Mn » pour masse moléculaire moyenne en nombre, « Mw » pour masse moléculaire moyenne en poids et « Ip » pour indice de polydispersité.

<u>Exemples</u>

<u>Exemple 1 : exemple de synthèse d'un copolymère statistique méthacrylate de méthyle/acide méthacrylique</u>

*1 ère étape : Synthèse du polymère*

Dans un réacteur à double enveloppe de 21, on place 1g de Trigonox 21 S (t-butylperoxy-2-éthylhexanoate) et 200 g de méthyléthyl cétone. Le mélange est chauffé au reflux pendant une heure. Après une heure, un mélange de 170 g de méthacrylate de méthyle et 30 g d'acide méthacrylique est ajouté au goutte à goutte sur une durée d'une heure. Le mélange incolore devient visqueux. Le chauffage est interrompu six heures après l'addition des monomères.

Composition par RMN: méthacrylate de méthyle 85,1%, acide méthacrylique 14,9 %.

Masse par GPC dans le tétrahydrofurane (THF) (standards polystyrène) : Mp = 98772 $g.mol^{-1}$ ; Mn = 61261

g.mol$^{-1}$ ; Mw= 105698 g.mol$^{-1}$ Ip=1,7.

*2ème étape : Mise en dispersion du polymère dans l'eau*

Au milieu réactionnel ci-dessus, on rajoute 200 g de méthyléthyl cétone et on chauffe à 60 °C. On ajoute goutte à goutte 30,86 g d'amino-2-méthyl-2-propanol pour neutraliser les fonctions acides et 1200 g d'eau. On évapore les solvants volatiles en chauffant jusqu'à 100°C. On obtient une dispersion aqueuse jaune transparente.

La température de transition vitreuse de ce copolymère, déterminée par DSC selon le protocole rapporté dans la description, est de 81 °C.

Exemple 2 : Composition cosmétique sous la forme d'une émulsion E/H/E

On a préparé la composition suivante :

| | |
|---|---|
| *Émulsion primaire (A)* : | |
| Eau | 10,20 g |
| Mélange de cétyl diméthicone copolyol, d'isostéarate de polyglycérol-4 et de laurate d'hexyle 40/30/30 (ABIL® WE 09 de Goldschmidt) | 3,50 g |
| Cyclopentasiloxane | 16,50 g |
| Dimethicone | 4,00 g |
| Copolymère éthylénique selon l'invention (exemple 1) | 65,00 g |
| Sulfate de Magnésium | 0,80 g |
| *Émulsion multiple* : | |
| Émulsion primaire (A) : | 22,50 g |
| Cyclopentasiloxane | 3,50 g |
| Huile d'amande d'abricot | 4,00 g |
| Eau | 68,05 g |
| Conservateurs | 1,00 g |
| Pentasodium éthylène diamine tétraméthylène phosphonate | 0,05 g |
| Copolymère acide acrylique/métacrylate de stéaryle (PEMULEN TR1® (Novéon)) | 0,60 g |
| Hydroxyde de sodium | 0,30 g |

**Mode Opératoire**

*Préparation de l'émulsion primaire:*

A température ambiante et sous agitation, on homogénéise l'Abil® WE 09, le cyclopentasiloxane et la dimethicone. Sous forte agitation, on incorpore lentement l'eau et le copolymère selon l'exemple 1 conforme à l'invention.

*Préparation de l'émulsion triple :*

A température ambiante et sous agitation, on disperse le copolymère d'alkylacrylate, les conservateurs et le séquestrant (pentasodium éthylène diamine tetramethylene phosphonate). On laisse gonfler environ 45 minutes sous agitation puis on neutralise avec l'hydroxyde de sodium. On dilue l'émulsion primaire avec le cyclopentasiloxane et l'huile d'amande d'abricot puis on incorpore ce mélange lentement sous agitation à la phase aqueuse.

Exemple 3 : Composition cosmétique sous la forme d'une émulsion E/H

On a préparé la composition suivante :

| | |
|---|---|
| A | |
| Cétyldiméthicone copolyol (ABIL® EM 90 de la société Goldschmidt) | 1,50 g |
| Isotearate polyglycerole | 0,50 g |
| Isohexadécane | 4,00 g |
| Squalane | 1,85 g |

(suite)

| | |
|---|---|
| A | |
| Dimethicone | 2,05 g |
| Huile d'amande d'abricot | 1,10 g |
| Cyclopentasiloxane | 9,00 g |
| Propylparabène | 0,15 g |
| B | |
| Eau | 30,70 g |
| Propylène glycol | 3,00 g |
| Sulfate de magnésium | 1,75 g |
| Méthylparabène | 0,20 g |
| Conservateur | 0,30 g |
| C | |
| Copolymère éthylénique selon l'invention (exemple 1) | 40,90 g |
| D | |
| Nylon 12 | 3,00 g |

Mode opératoire :

On homogénéise à température ambiante sous agitation la phase A et la phase B séparément.

On réalise l'émulsion en incorporant la phase B dans la phase A.

On incorpore les phases C et D sous agitation.

**RESULTATS:**

**Test de stabilité**

On a étudié la stabilité après deux mois de conservation à 4 °C, 25 °C, 37 °C et 45 °C des compositions des Exemples 2 et 3 ci-dessus (renfermant respectivement 7 % et 2,5 % en poids, en matière active, de copolymère tenseur conforme à l'invention. Les résultats sont rassemblés dans le Tableau ci-dessous :

| **Compositions** | Stabilité |
|---|---|
| Exemple 2 : Emulsion E/H/E contenant 2,5% en matière active, de copolymère selon l'exemple 1. | oui |
| Exemple 3 : Emulsion E/H contenant 7% en matière active, de copolymère selon l'exemple 1. | oui |

Il ressort du Tableau ci-dessus que les compositions selon l'invention (exemple 2 et 3) sont stables et ce à toutes températures de stockage.

**Revendications**

1. Composition cosmétique comprenant, dans un milieu physiologiquement acceptable, au moins un copolymère statistique à chaîne principale linéaire de nature éthylénique, dans laquelle ledit copolymère :

   (i) présente une masse moléculaire comprise entre 15 000 et 600 000 g/mol,
   (ii) contient au moins 70 % d'unités monomères issues de monomères dont les homopolymères sont hydrophobes et présentent une température de transition vitreuse supérieure à 40 °C,
   (iii) contient également au moins une « unité monomère » issue d'un monomère hydrophile ionique au moins partiellement neutralisée, et
   (iv) présente une température globale de transition vitreuse supérieure ou égale à 45 °C

   et se présentant sous la forme d'une émulsion eau-dans-huile ou d'une émulsion multiple.

2. Composition selon la revendication 1, dans laquelle le copolymère comprend plus de 75 %, de préférence plus de

80 %, en poids par rapport à son poids total d'« unités monomères » issues de monomères dont les homopolymères présentent une température de transition vitreuse supérieure à 40 °C, et jusqu'à 95 % en poids.

**3.** Composition selon l'une quelconque des revendications 1 à 2, dans laquelle les monomères dont les homopolymères présentent une température de transition vitreuse supérieure à 40 °C sont choisi parmi les composés vinyliques, les acrylates, les méthacrylates et les (méth)acrylamides.

**4.** Composition selon l'une quelconque des revendications 1 à 3, dans laquelle les monomères dont les homopolymères présentent une température de transition vitreuse supérieure à 40 °C sont choisi parmi :

- les composés vinyliques de formule

$$CH_2 = CHR_1,$$

où $R_1$ est un groupe

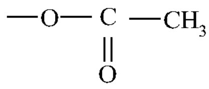

,

un groupe cycloalkyle en $C_3$ à $C_8$ ou un groupe aryle en $C_6$ à $C_{20}$,
- les acrylates de formule

$$CH_2 = CHCOOR_2$$

où $R_2$ est un groupe tertiobutyle, un groupe cycloalkyle en $C_3$ à $C_8$ éventuellement ponté par un groupe alkylène en $C_1$ à $C_4$, ou un groupe aralkyle en $C_7$ à $C_{30}$ (groupe alkyle en $C_1$ à $C_4$),
- les méthacrylates de formule

$$CH_2 = C(CH_3)\ COOR_3$$

où $R_3$ est un groupe isobutyle, tertiobutyle, un groupe alkyle en $C_1$ à $C_3$ linéaire ou ramifié, un groupe cycloalkyle en $C_3$ à $C_8$ éventuellement ponté par un groupe alkylène en $C_1$ à $C_4$ ou un groupe aralkyle en $C_7$ à $C_{30}$ (groupe alkyle en $C_1$ à $C_4$), et
- les (méth)acrylamides de formule

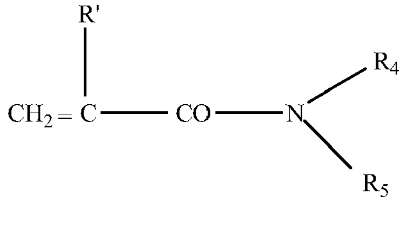

où R' désigne H ou $-CH_3$, et où $R_4$ et $R_5$ identiques ou différents représentent chacun un atome d'hydrogène ou un groupe alkyle linéaire ou ramifié en $C_4$ à $C_{12}$, étant entendu que $R_4$ et $R_5$ ne peuvent représenter simultanément un atome d'hydrogène.

**5.** Composition selon l'une quelconque des revendications 1 à 4, dans laquelle les monomères dont les homopolymères présentent une température de transition vitreuse supérieure à 40 °C sont choisis parmi le styrène, l'acrylate de benzyle, l'acrylate de cycloalkyle en $C_3$ à $C_8$ éventuellement ponté par un groupe alkylène en $C_1$ à $C_4$, l'acrylate de tertiobutyle, le méthacrylate de $(C_1\text{-}C_3)$alkyle, le méthacrylate de tertiobutyle, le méthacrylate de benzyle et le méthacrylate de cycloalkyle en $C_3$ à $C_8$ éventuellement ponté par un groupe alkylène en $C_1$ à $C_4$.

**6.** Composition selon l'une quelconque des revendications 1 à 5, dans laquelle les monomères dont les homopolymères

présentent une température de transition vitreuse supérieure à 40 °C sont choisis parmi le vinylcyclohexane, le styrène, l'acétate de vinyle, l'acrylate de benzyle, l'acrylate de cyclohexyle, l'acrylate de tertiobutyle, l'acrylate d'isobornyle et l'acrylate de norbornyle, le méthacrylate de méthyle, d'éthyle, d'isobutyle, de cyclohexyle, de benzyle, de tertiobutyle, d'isobornyle et de norbornyle, le N-butylacrylamide, le N-t-butylacrylamide et le N,N-dibutylacrylamide.

**7.** Composition selon l'une quelconque des revendications 1 à 6, dans laquelle les monomères dont les homopolymères présentent une température de transition vitreuse supérieure à 40 °C sont choisis parmi l'acrylate de benzyle, l'acrylate de cyclohexyle, l'acrylate de tertiobutyle, l'acrylate d'isobornyle et l'acrylate de norbornyle, le méthacrylate de méthyle d'éthyle, d'isobutyle, de cyclohexyle, de benzyle, de teriobutyle, d'isobornyle et de norbornyle et le styrène.

**8.** Composition selon l'une quelconque des revendications 1 à 7, dans laquelle les monomères dont les homopolymères présentent une température de transition vitreuse supérieure à 40 °C sont choisis parmi le méthacrylate de méthyle et le méthacrylate de cyclohexyle.

**9.** Composition selon l'une quelconque des revendications 1 à 8, dans laquelle le monomère hydrophile ionique est choisi parmi les monomères hydrophiles anioniques, les monomères hydrophiles cationiques, les monomères amphotères et leurs mélanges.

**10.** Composition selon la revendication 9, dans laquelle le monomère hydrophile anionique est défini par la formule (I)

$$CH_2{=}CR_6\,(Z)_n\,(R_7)_m\,Y \qquad (I)$$

où

- Z prend l'une des significations suivantes : C(=O)O, C(=O)NH, O, O(C=O),
- n est égal à 0 ou 1,
- m est égal à 0 ou 1,
- $R_6$ est un atome d'hydrogène, un groupe $CH_3$, ou un groupe ($C_2$-$C_{30}$)alkyle,
- $R_7$ est choisi parmi les groupements alkylènes linéaires saturés ou non et/ou branchés et/ou cycliques (aromatiques ou non) en $C_1$ à $C_{30}$, incluant ou non un ou plusieurs hétéroatomes, et
- Y est choisi parmi les groupes suivants : -COOH, $-SO_3H$, $-OSO_3H$, $-OP(OH)_2$, $-OPO(OH)_2$.

**11.** Composition selon la revendication 10, dans laquelle $R_6$ est un atome d'hydrogène, un groupe $CH_3$ ou un $C_2H_5$ et $R_7$ est choisi parmi les groupes alkylène en $C_1$ à $C_{30}$, phénylène, benzylène, $-(CH_2\text{-}CH{=}CH)-$ et (CHOH).

**12.** Composition selon l'une quelconque des revendications 9 à 11, dans laquelle le monomère hydrophile anionique est choisi parmi :

- les monomères à insaturation(s) éthylénique(s) comprenant au moins une fonction acide carboxylique (COOH), phosphonique ($PO_3H_2$) ou sulfonique ($SO_3H$), comme par exemple l'acide acrylique, l'acide méthacrylique, le (méth)acrylate de 2-carboxyéthyle, l'acide vinylbenzoïque, l'acide acrylamidoglycolique $CH_2{=}CHCONHCH(OH)CO_2H$, l'acide maléique, l'acide acrylamidopropanesulfonique, l'acide acrylamido 2-méthylpropanesulfonique, l'acide styrène sulfonique, l'acide vinylsulfonique, le méthacrylate ou l'acrylate d'acide propyl-3-sulfonique ($CH_2{=}C(CH_3)CO_2(CH_2)_3SO_3H$), le méthacrylate ou l'acrylate d'acide éthyl-2-sulfonique ($CH_2{=}C(CH_3)CO_2(CH_2)_2SO_3H$) et la vinylméthylsulfone, l'acide vinylphosphonique ($CH_2{=}CH\text{-}PO(OH)_2$), le méthacrylate d'acide éthyl-2-phosphonique ($CH_2{=}C(CH_3)COOCH_2CH_2OP(O)(OH)_2$),
- les anhydrides carboxyliques porteurs d'une liaison vinylique tels que l'anhydride maléique,
- les diacides tels que l'acide crotonique, l'acide itaconique, l'acide fumarique, l'acide maléique,
- ainsi que leurs mélanges.

**13.** Composition selon l'une quelconque des revendications 1 à 12, dans laquelle le monomère hydrophile est l'acide (méth)acrylique.

**14.** Composition selon l'une quelconque des revendications 1 à 13, dans laquelle la teneur en poids d'« unités monomères » issues de monomères hydrophiles ioniques dans le copolymère est comprise entre 5 et 30 % préférentiellement entre 10 et 25 % et de façon encore plus préférée entre 10 et 20 % par rapport au poids total du copolymère.

**15.** Composition selon l'une quelconque des revendications 1 à 14, dans laquelle le copolymère est choisi parmi

- Les copolymères de méthacrylate de méthyle / acide méthacrylique ; les copolymères de méthacrylate de méthyle / acide acrylique, lesdits copolymères contenant entre 70 et 90 % en poids de méthacrylate de méthyle ;
- Les copolymères de méthacrylate d'éthyle/ acide méthacrylique ; les copolymères de méthacrylate d'éthyle / acide acrylique, lesdits copolymères contenant entre 70 et 90 % en poids de méthacrylate d'éthyle ;
- Les copolymères de méthacrylate d'isobutyle/ acide méthacrylique ; les copolymères de méthacrylate d'isobutyle / acide acrylique, lesdits copolymères contenant entre 70 et 90 % en poids de méthacrylate d'isobutyle ;
- Les copolymères de méthacrylate de benzyle / acide méthacrylique ; les copolymères de méthacrylate de benzyle / acide acrylique, lesdits copolymères contenant entre 70 et 90 % en poids de méthacrylate de benzyle ;
- Les copolymères d'acrylate de benzyle / acide méthacrylique ; les copolymères d'acrylate de benzyle / acide acrylique, lesdits copolymères contenant entre 70 et 90 % en poids d'acrylate de benzyle ;
- Les copolymères de méthacrylate de cyclohexyle / acide méthacrylique ; les copolymères de méthacrylate de cyclohexyle / acide acrylique, lesdits copolymères contenant entre 70 et 90 % en poids de méthacrylate de cyclohexyle ;
- Les copolymères d'acrylate de cyclohexyle / acide méthacrylique ; les copolymères d'acrylate de cyclohexyle / acide acrylique, lesdits copolymères contenant entre 70 et 90 % en poids d'acrylate de cyclohexyle ;
- Les copolymères de méthacrylate de tertio-butyle/ acide méthacrylique ; les copolymères de méthacrylate de tertio-butyle / acide acrylique, lesdits copolymères contenant entre 70 et 90 % en poids de méthacrylate de tertio-butyle ;
- Les copolymères d'acrylate de tertio-butyle/ acide méthacrylique ; les copolymères d'acrylate de tertio-butyle / acide acrylique, lesdits copolymères contenant entre 70 et 90 % en poids d'acrylate de tertio-butyle ;
- Les copolymères de méthacrylate d'isobornyle/ acide méthacrylique ; les copolymères de méthacrylate d'isobornyle / acide acrylique, lesdits copolymères contenant entre 70 et 90 % en poids de méthacrylate d'isobornyle ;
- Les copolymères d'acrylate d'isobornyle / acide méthacrylique ; les copolymères d'acrylate d'isobornyle / acide acrylique, lesdits copolymères contenant entre 70 et 90 % en poids d'acrylate d'isobornyle ;
- Les copolymères de méthacrylate de norbornyle/ acide méthacrylique ; les copolymères de méthacrylate de norbornyle / acide acrylique, lesdits copolymères contenant entre 70 et 90 % en poids de méthacrylate de norbornyle ;
- Les copolymères d'acrylate de norbornyle / acide méthacrylique ; les copolymères d'acrylate de norbornyle / acide acrylique, lesdits copolymères contenant entre 70 et 90 % en poids d'acrylate de norbornyle et ;
- Les copolymères de styrène / acide méthacrylique ; les copolymères de styrène / acide acrylique, lesdits copolymères contenant entre 70 et 90 % en poids de styrène.

**16.** Composition selon l'une quelconque des revendications 1 à 15, dans laquelle le copolymère comprend en outre au moins une « unité monomère » issue d'un monomère dont l'homopolymère présente une température de transition vitreuse inférieure à 40 °C.

**17.** Composition selon la revendication 16, dans laquelle le monomère dont l'homopolymère présente une température de transition vitreuse inférieure à 40 °C est présent en une teneur inférieure ou égale à 25 % en poids, de préférence de 5 à 25 % en poids, par rapport au poids total du copolymère, et de préférence en une teneur inférieure ou égale à 10 % en poids, de préférence de 5 à 10 % en poids.

**18.** Composition selon l'une quelconque des revendications 1 à 17, dans laquelle le copolymère tel que défini dans l'une quelconque des revendications 1 à 17 est compris dans une teneur exprimée en matière sèche allant de 0,1 à 15 %, de préférence de 1 à 10 % et de façon encore plus préférée de 1 à 5 % en poids par rapport au poids total de la composition.

**19.** Composition selon l'une quelconque des revendications précédentes se présentant sous la forme d'une émulsion eau-dans-huile comprenant une phase aqueuse interne et une phase grasse comprenant au moins une huile et au moins un tensioactif.

**20.** Composition selon la revendication 19, dans laquelle le tensioactif est choisi parmi les tensioactifs siliconés, les tensioactifs polyisobutylene à terminaison succinique estérifiée et les élastomères de silicone émulsionnants et leurs mélanges.

**21.** Composition selon la revendication 19, dans laquelle la phase aqueuse interne est non gélifiée.

**22.** Composition selon l'une quelconque des revendications 20 ou 21, dans laquelle ledit tensioactif siliconé est choisi parmi les alkyldiméthicones copolyols et les diméthicones copolyols.

**23.** Composition selon la revendication précédente, dans laquelle le tensioactif siliconé est choisi parmi le mélange de cyclométhicone et de diméthicone copolyol, le laurylméthicone copolyol, le cétyl diméthicone copolyol, le mélange polyglycéryl-4 isostéarate / cétyl diméthicone copolyol / hexyllaurate et leurs mélanges.

**24.** Composition selon l'une quelconque des revendications 19 à 23, dans laquelle la phase grasse comprend au moins une huile de silicone.

**25.** Composition selon l'une quelconque des revendications 19 à 24, comprenant en outre au moins un co-émulsionnant.

**26.** Composition selon la revendication précédente, dans laquelle ledit co-émulsionnant est un ester alkylé de polyol.

**27.** Composition selon la revendication précédente, dans laquelle l'ester alkylé de polyol est un ester de glycérol et/ou de sorbitane choisi parmi l'isostérate de polyglycérol, l'isostérate de sorbitane, l'isostérate de sorbitane et de glycérol, et leurs mélanges.

**28.** Composition selon l'une quelconque des revendications 1 à 18, se présentant sous la forme d'une émulsion multiple eau-dans-huile-dans-eau.

**29.** Composition selon la revendication précédente, se présentant sous la forme d'une émulsion multiple obtenue par émulsification d'une émulsion eau-dans-huile telle que définie dans les revendications 20 à 28 dans une phase aqueuse gélifiée.

**30.** Composition selon la revendication 29, dans laquelle la phase aqueuse externe contient au moins un copolymère tensioactif constitué d'une fraction majoritaire d'un monomère d'acide carboxylique monooléfiniquement insaturé en $C_3$-$C_6$ ou de son anhydride et d'une fraction minoritaire de monomère ester gras d'acide acrylique.

**31.** Composition selon la revendication précédente, dans laquelle le copolymère tensioactif est un copolymère acrylate/$C_{10}$-$C_{30}$ alkylacrylate.

**32.** Composition selon la revendication 29, dans laquelle l'une des phases aqueuses a une valeur d'activité en eau inférieure ou égale à 0,85.

**33.** Utilisation comme agent tenseur, dans une composition cosmétique sous forme d'émulsion eau-dans-huile ou d'émulsion multiple, d'au moins un copolymère tel que défini selon l'une quelconque des revendications 1 à 17.

**34.** Procédé de traitement cosmétique d'une peau vieillie, notamment ridée, comprenant l'application sur ladite peau d'au moins une composition selon l'une quelconque des revendications 1 à 32, en une quantité efficace pour estomper les rides par un effet tenseur.

**35.** Produit cosmétique comprenant au moins (i) une première composition, sous la forme d'une émulsion eau-dans-huile ou d'une émulsion multiple, contenant au moins un copolymère tel que défini selon l'une quelconque des revendications 1 à 17 et (ii) une deuxième composition comprenant au moins un milieu physiologiquement acceptable.

**36.** Produit selon la revendication précédente, dans lequel la première composition est telle que définie dans l'une quelconque des revendications 1 à 32.

**37.** Kit de compositions comprenant un produit selon la revendication 35 ou 36.

**38.** Kit selon la revendication 37, dans lequel les première et seconde compositions sont conditionnées dans des compartiments ou récipients distincts.

**39.** Kit selon la revendication 38, dans lequel lesdites compositions sont conditionnées sous la forme d'un ensemble de distribution double pompe.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 9829092 A
- EP 1038519 A
- WO 0030595 A
- FR 2843025
- FR 2822676
- FR 2792190 A
- FR 2811565
- WO 04100904 A
- US 5236986 A
- US 5412004 A
- US 5837793 A
- US 5811487 A
- EP 0648102 A
- EP 0268164 A
- EP 0908170 A
- EP 0507693 A
- EP 0779071 A
- US 5692644 A
- US 5884759 A
- US 5875888 A
- US 5875889 A
- US 5992693 A
- US 5944175 A
- US 6402364 B

### Littérature non-brevet citée dans la description

- **KIRK-OTHMER.** Encyclopedia of Chemical Technology. WILEY, 1979, vol. 22, 333-432
- **F. PUISIEUX ; M. SEILLER.** Les Systèmes Dispersés-I Agents de Surface et Emulsions. 153-155